# EUROPÄISCHE PATENTSCHRIFT

(11) **EP 0 793 496 B1**
(45) Veröffentlichungstag und Bekanntmachung des Hinweises auf die Patenterteilung: **05.07.2000**
(21) Anmeldenummer: 95936988.5
(22) Anmeldetag: 27.11.1995
(51) Int. Cl.: A61K 31/435

(54) **VERWENDUNG HALOGENIERTER beta-CARBOLINE ALS MODELLSUBSTANZEN ZUR ERZEUGUNG NEURODEGENERATIVER ERKRANKUNGEN**
USE OF HALOGENATED beta-CARBOLINES AS MODEL SUBSTANCES TO PRODUCE NEURODEGENERATIVE DISORDERS
UTILISATION DE beta-CARBOLINES HALOGENEES EN TANT QUE SUBSTANCES EXPERIMENTALES SERVANT A PROVOQUER DES AFFECTIONS DEGENERATIVES

(30) Priorität: 25.11.1994 DE 4442097
(43) Veröffentlichungstag der Anmeldung: 10.09.1997
(73) Patentinhaber: Bringmann, Gerhard, 97074 Würzburg (DE); Wesemann, Wolfgang, 35033 Marburg (DE); Sontag, Karl-Heinz, 37154 Northeim (DE); Riederer, Peter, 97080 Würzburg (DE); Rausch, Wolf-Dieter, 1030 Wien (AT); Feineis, Doris, 97074 Würzburg (DE); God, Ralf, 97074 Würzburg (DE); Clement, Hans-Willi, 35033 Marburg (DE); Grote, Christoph, 35033 Marburg (DE); Gsell, Wieland, 97080 Würzburg (DE); Götz, Mario E., 97080 Würzburg (DE); Zielke, Barbara, 97080 Würzburg (DE)
(72) Erfinder: Bringmann, Gerhard, 97074 Würzburg (DE); Wesemann, Wolfgang, 35033 Marburg (DE); Sontag, Karl-Heinz, 37154 Northeim (DE); Riederer, Peter, 97080 Würzburg (DE); Rausch, Wolf-Dieter, 1030 Wien (AT); Feineis, Doris, 97074 Würzburg (DE); God, Ralf, 97074 Würzburg (DE); Clement, Hans-Willi, 35033 Marburg (DE); Grote, Christoph, 35033 Marburg (DE); Gsell, Wieland, 97080 Würzburg (DE); Götz, Mario E., 97080 Würzburg (DE); Zielke, Barbara, 97080 Würzburg (DE)
(74) Vertreter: Schüssler, Andrea, Dr.
(86) Internationale Anmeldenummer: DE9501675
(87) Internationale Veröffentlichungsnummer: WO9616651

(56) Entgegenhaltungen:
- JOURNAL OF NEURAL TRANSMISSION, Bd. 46, Nr. suppl., 1995, Seiten 235-244, XP000578428 BRINGMANN, G. ET AL: "The TaClo concept: 1-trichloromethyl-1,2,3,4-tetrahydro-beta- carboline (TaClo), a new toxin for dopaminergic neurons"
- JOURNAL OF NEURAL TRANSMISSION, Bd. 46, Nr. suppl., 1995, Seiten 283-289, XP000578423 SONTAG, K.-H. ET AL: "Long-term behavioural effects of TaClo (1-trichloromethyl-1,2,3,4-tetrahydro-beta -carboline) after subchronic treatment in rats"
- JOURNAL OF NEURAL TRANSMISSION, Bd. 46, Nr. suppl., 1995, Seiten 275-281, XP000578424 GROTE, C. ET AL: "Biochemical lesions of the nigrostriatal system by TaClo (1-trichloromethyl-1,2,3,4-tetrahydro-beta -carboline) and derivatives"
- JOURNAL OF NEURAL TRANSMISSION, Bd. 38, Nr. suppl., 1992, Seiten 15-26, XP000578706 BRINGMANN, G. ET AL: "Trichloroharmanes as potential endogenously formed inducers of Morbus Parkinson: synthesis, analytics, and first in vivo-investigations"
- PROGRESS IN NEUROPSYCHOPHARMACOLOGY AND BIOLOGICAL PSYCHIATRY, Bd. 17, Nr. 3, 1993, Seiten 487-499, XP000578714 WESEMANN, W. ET AL: "Functional studies on monoaminergic transmitter release in Parkinsonism"
- NEUROSCIENCE LETTERS, Bd. 105, 1989, Seiten 344-349, XP000578710 NEAFSEY, E.J. ET AL: "Striatal dopamine toxicity following intranigral injection in rats of 2-methyl-norharman, a beta-carbolinium analog of N-methyl-4-phenylpyridinium ion (MPP+)"
- BRAIN RESEARCH, Bd. 570, 1992, Seiten 154-160, XP000578721 COLLINS, M.A. ET AL: "Indole-N-methylated beta-carbolinium ions as potential brain-bioactivated neurotoxins"

## Beschreibung

Die vorliegende Erfindung betrifft die Verwendung halogenierter β-Carboline zur Erzeugung neurodegenerativer Erkrankungen.

Weltweit beschäftigen sich Wissenschaftler intensiv mit der Hypothese, daß auch Umweltfaktoren bei der Pathogenese neurodegenerativer Erkrankungen wie z.B. des Morbus Parkinson oder des Morbus Alzheimer eine wesentliche Rolle spielen könnten. Deutlichen Auftrieb erfuhr diese Vermutung vor etwa einem Jahrzehnt, als man bei Drogenabhängigen, die sich eine mit 1-Methyl-4-phenyl-1,2,3,6-tetrahydropyridin (MPTP) verunreinigte Droge injiziert hatten, ein schweres irreversibles Parkinsonsyndrom beobachtete (Langston et al., 1983; Tanner 1989). Dadurch wurde eine intensive Suche nach weiteren neurotoxisch wirkenden Substanzen ausgelöst, mit denen der Mensch natürlicherweise durch exogene Zufuhr (Nahrung, Umwelt) bzw. durch endogene Bildung in Berührung kommt. Als strukturell und wirkungsmäßig ähnlich zu MPTP werden die Trichlorharmane eingestuft, die von Bringmann et al., J. Neural. Transm. (1992), [Suppl.] 38, S. 15-26 beschrieben wurden.

Die Aufgabe der vorliegenden Erfindung bestand deshalb darin, weitere neurotoxisch wirkende Substanzen zu finden, so daß mit deren Hilfe das Entstehen und die Ursachen neurodegenerativer Erkrankungen aufgeklärt werden kann.

In diesem Zusammenhang wurden β-Carboline (Harmane) untersucht, die im Säugerorganismus vermutlich durch nicht-enzymatische Pictet-Spengler-Kondensation von Indolylethylaminen mit Carbonylverbindungen wie Formaldehyd oder Acetaldehyd gebildet werden können und neuropharmakologisches Potential zeigen.

Die in der vorliegenden Anmeldung beschriebene Substanzgruppe hochchlorierter β-Carboline mit neurotoxischer Potenz kommt normalerweise nicht im Organismus vor. Jedoch immer wenn Chloral im Organismus nachweisbar ist, z.B. nach medikamentöser Gabe von Chloralhydrat oder nach Inkorporation des Lösungsmittels Trichlorethylen, muß unter Berücksichtigung der Reaktivität des Chlorals gegenüber biogenen oder medikamentös verabreichten Aminen wie z.B. Dopamin, Tryptamin, usw. mit der Entstehung von chlorierten β-Carbolinen gerechnet werden.

Für die Substanzgruppe der halogenierten β-Carboline konnte nun erstmals eine ausgeprägte neuropharmakologische Aktivität nachgewiesen werden: Verhaltenstests und biochemische Untersuchungen mit ausgewählten synthetisch hergestellten Vertretern zeigen u.a. aufgrund der Beeinflussung des Neurotransmitter-Stoffwechsels, der Inhibierung der Atmungskettenenzyme sowie histochemischer Untersuchungen, daß solche Verbindungen als potente neurotoxisch wirkende Substanzen einzustufen sind. 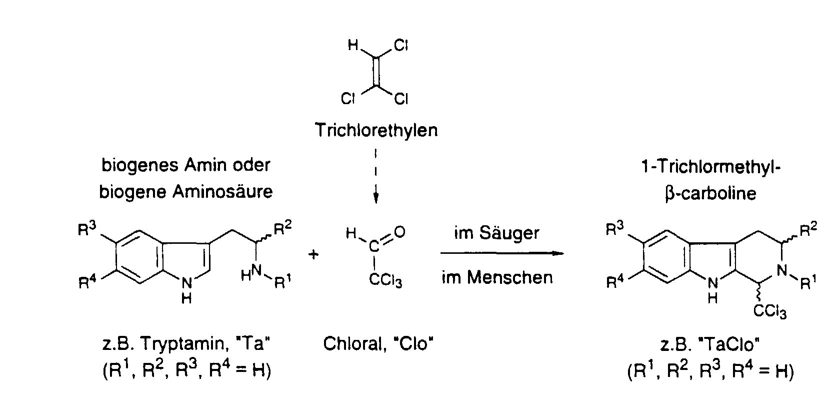 Mögliche Entstehung von 1-Trichlormethyl-β-carbolinen.

Es gelang den Erfindern der Nachweis, daß diese Substanzen, wie z.B. TaClo, tatsächlich im lebenden Organismus entstehen können. Dies konnte mit dem folgenden Tierexperiment eindrucksvoll gezeigt werden: Wird über einen längeren Zeitraum Tryptamin und Chloralhydrat an Ratten verabreicht, dann zeigen die Tiere Auffälligkeiten in ihrer lokomotorischen Nachtaktivität. Diese ist signifikant erniedrigt und zeigt eine anomale Rhythmik. Da diese spontane Aktivität positiv mit dem Dopaminstoffwechsel korreliert ist (O'Neill und Fillenz, 1985), deutet dieser Befund darauf hin, daß ein dopaminerges Neurotoxin wie z.B. TaClo gebildet wurde. Durch gaschromatographisch-massenspektrometrische Methoden (GC/MSD) konnte nun tatsächlich in Blut- und Gehirnproben dieser Tiere der endogen gebildete Heterocyclus, TaClo, identifiziert und eindeutig charakterisiert werden.

Es gelang den Erfindern, die halogenierten β-Carbolin-Derivate auch synthetisch bereitzustellen. Diese β-Carbolin-Derivate haben die allgemeine Hauptgrundstruktur (I):

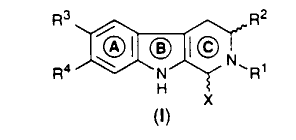

Im Ring C können dabei eine, zwei oder drei Doppelbindungen vorhanden sein.

In der Formel (**I**) kann X -C(Hal)₃, -CH(Hal)₂, -CH₂(Hal) oder eine =C(Hal)₂-Gruppe mit Hal = F, Cl, Br, I sein.

Die Substituenten R¹, R², R³ und R⁴ haben dabei folgende Bedeutungen:
R¹ kann -H, eine C₁-C₁₈ Alkylgruppe (z.B. Methyl, Ethyl, Propyl, *n*-Butyl, *iso*-Butyl, *tert*.-Butyl usw.), -CH₂-CH=CH₂, -CH₂-C₆H₅, eine Acylgruppe (z.B. Formyl, Acetyl, Monochloracetyl, Trichloracetyl usw.) oder Aroylgruppe sein, mit der Maßgabe, daß R¹ auch ein freies Elektronenpaar sein kann, wenn der Stickstoff an der Ausbildung einer Doppelbindung beteiligt ist,
R² kann -H, -COOH, -COOCH₃, -COOC₂H₅, -COOCH₂-CH=CH₂, -COOCH₂-C₆H₅, -COONH₂, -CO₂NR₂, -CH₂OAc, -CH₂-OH, etc. sein,
R³ kann H-, HO-, CH₃O-, C₂H₅O-, C₆H₅-CH₂O-, CH₃COO-, C₂H₅COO-, etc. sein,
R⁴ kann H-, HO-, CH₃O-, C₂H₅O-, C₆H₅-CH₂O-, CH₃COO-, C₂H₅COO-, etc. sein.

Es ergeben sich bevorzugte Untergrundstrukturen:

### Grundstruktur (1)

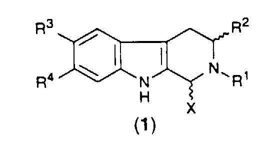

bevorzugt
X = -CCl₃, R¹ = -C₂H₅, R²-R⁴ = -H
X = -CCl₃, R¹ = -COCH₂Cl, R²-R⁴ = -H
X = -CCl₃, R¹ = -COCCl₃, R²-R⁴ = -H
X = -CCl₃, R¹-R² = -H, R³ = -OH, R⁴ = -H
X = -CCl₃, R¹-R² = -H, R³ = -OCH₃, R⁴ = -H
X = -CCl₃, R¹-R² = -H, R³ = -H, R⁴ = -OH
X = -CCl₃, R¹-R² = -H, R³ = -H, R⁴ = -OCH₃
X = -CBr₃, R¹-R⁴ = -H
X = -CBr₃, R¹ = -CHO, R²-R⁴ = -H
ganz bevorzugt
X = -CCl₃, R¹ = -CH₃, R²-R⁴ = -H
X = -CCl₃, R¹ = -CH₂-C₆H₅, R²-R⁴ = -H
X = -CCl₃, R¹ = -CH₂-C₆H₅, R² = -COOCH₃, R³-R⁴ = -H
X = -CCl₃, R¹ = -CH₃, R² = -COOCH₃, R³-R⁴ = -H

### Grundstruktur (2)

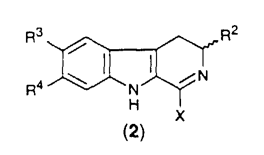

bevorzugt
X = -CF₃, R²-R⁴ = -H
X = -CCl₃, R²-R⁴ = -H
X = -CBr₃, R²-R⁴ = -H
ganz bevorzugt
X = -CF₃, R² = -COOCH₃, R³-R⁴ = -H
X = -CCl₃, R² = -COOCH₃, R³-R⁴ = -H
X = -CBr₃, R² = -COOCH₃, R³-R⁴ = -H

### Grundstruktur (3)

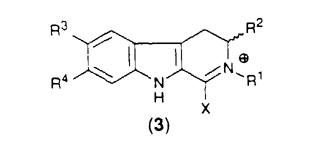

bevorzugt
X = -CCl₃, R¹ = -C₂H₅, R²-R⁴ = -H
X = -CCl₃, R¹ = -CH₃, R²-R⁴ = -H
X = -CBr₃, R¹ = -C₂H₅, R²-R⁴ = -H
X = -CBr₃, R¹ = -CH₃, R²-R⁴ = -H
ganz bevorzugt
X = -CCl₃, R¹ = -C₂H₅, R² = -COOCH₃, R³-R⁴ = -H
X = -CCl₃, R¹ = -CH₃, R² = -COOCH₃, R³-R⁴ = -H
X = -CBr₃, R¹ = -C₂H₅, R² = -COOCH₃, R³-R⁴ = -H
X = -CBr₃, R¹ = -CH₃, R² = -COOCH₃, R³-R⁴ = -H

### Grundstruktur (4)

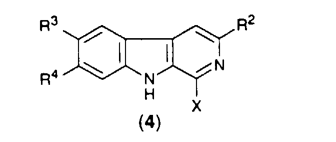

bevorzugt
X = -CCl₃, R²-R⁴ = -H
X = -CBr₃, R²-R⁴ = -H
ganz bevorzugt
X = -CCl₃, R² = -COOCH₃, R³-R⁴ = -H
X = -CBr₃, R² = -COOCH₃, R³-R⁴ = -H

### Grundstruktur (5)

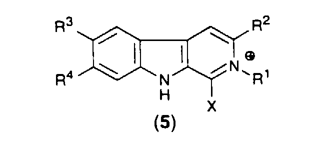

bevorzugt
X = -CCl₃, R¹ = -CH₃, R²-R⁴ = -H
X = -CCl₃, R¹ = -C₂H₅, R²-R⁴ = -H
X = -CCl₃, R¹ = -CH₂-C₆H₅, R²-R⁴ = -H
X = -CBr₃, R¹ = -CH₃, R²-R⁴ = -H
X = -CBr₃, R¹ = -C₂H₅, R²-R⁴ = -H
X = -CBr₃, R¹ = -CH₂-C₆H₅, R²-R⁴ = -H
ganz bevorzugt
X = -CCl₃, R¹ = -CH₃, R² = -COOCH₃, R³-R⁴ = -H
X = -CCl₃, R¹ = -C₂H₅, R² = -COOCH₃, R³-R⁴ = -H
X = -CCl₃, R¹ = -CH₂-C₆H₅, R² = -COOCH₃, R³-R⁴ = -H
X = -CBr₃, R¹ = -CH₃, R² = -COOCH₃, R³-R⁴ = -H
X = -CBr₃, R¹ = -C₂H₅, R² = -COOCH₃, R³-R⁴ = -H
X = -CBr₃, R¹ = -CH₂-C₆H₅, R² = -COOCH₃, R³-R⁴ = -H

Weitere Derivate mit beliebigen Substituenten in 4-, 5- und 8-Position des β-Carbolingerüstes sind eingeschlossen, wobei die Nummerierung des β-Carbolingerüstes wie folgt ist:

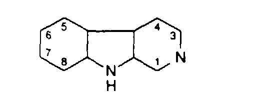

Ebenfalls eingeschlossen sind Derivate bei denen der Stickstoff im Indolring alkyliert (z.B. -CH₃, -C₂H₅, -C₃H₇, etc.) oder acyliert (z.B. -COCH₃, -COC₂H₅, etc.) ist.

Besonders bevorzugt sind Verbindungen mit der folgenden Formel: 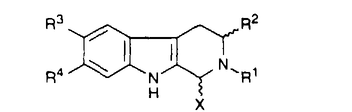
R¹ = Alkyl mit C₁-C₁₈, z.B. Methyl, Ethyl, Propyl, *n*-Butyl, *iso*-Butyl, *tert*.-Butyl etc.
R² - R⁴ und X haben die oben bei der Struktur (**I**) definierten Bedeutungen

Davon sind besonders bevorzugt:
X = -CCl₃, R¹ = -CH₃, R²-R⁴ = -H
X = -CCl₃, R¹ = -C₂H₅, R²-R⁴ = -H
X = -CCl₃, R¹ = -CH₃, R² = COOCH₃, R³-R⁴ = -H
X = -CCl₃, R¹ = -C₂H₅, R² = COOCH₃, R³-R⁴ = -H
X = -CBr₃, R¹ = -CH₃, R²-R⁴ = -H
X = -CBr₃, R¹ = -C₂H₅, R²-R⁴ = -H
X = -CBr₃, R¹ = -CH₃, R² = COOCH₃, R³-R⁴ = -H
X = -CBr₃, R¹ = -C₂H₅, R² = COOCH₃, R³-R⁴ = -H

Auch Verbindungen mit der folgenden Formel sind bevorzugt:

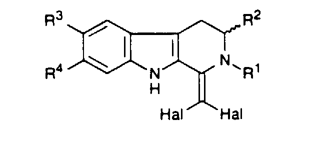

Hal hat dabei die Bedeutung von F, Cl, Br oder I, die gleich oder verschieden sein können.

Die Substituenten R¹, R², R³ und R⁴ haben die bei der Struktur (**I**) definierten Bedeutungen.

Davon sind besonders bevorzugt:
Hal = -Cl, R¹ -R⁴ = -H
Hal = -Cl, R¹ = -CH₃, R²-R⁴ = -H
Hal = -Cl, R¹ = -H, R² = -COOCH₃, R³-R⁴ = -H
Hal = -Cl, R¹ = -CH₃, R² = -COOCH₃, R³-R⁴ = -H
Hal = -Br, R¹ -R⁴ = -H
Hal = -Br, R¹ = -CH₃, R²-R⁴ = -H
Hal = -Br, R¹ = -CHO, R²-R⁴ = -H
Hal = -Br, R¹ = -H, R² = -COOCH₃, R³-R⁴ = -H
Hal = -Br, R¹ = -CH₃, R² = -COOCH₃, R³-R⁴ = -H

Aus der Vielzahl der durch die Grundstrukturen abgedeckten Verbindungen seien nun weitere genannt, die sich durch eine besonders gute Wirksamkeit ausgezeichnet haben, wobei hervorgehoben werden muß, daß alle unter die Grundstrukturen fallenden Verbindungen die oben genannte Aufgabe lösen:
Grundstruktur (**1**), X = -CCl₃, R¹ = -CH₂-CH=CH₂, R²-R⁴ = -H
Grundstruktur (**1**), X = -CCl₃, R¹-R⁴ = -H, Methylgruppe in 8-Position
Grundstruktur (**1**), X = -HCCl₂, R¹-R⁴ = -H
Grundstruktur (**1**), X = -HCCl₂, R¹ = -COCCl₃, R²-R⁴ = -H
Grundstruktur (**1**), X = -HCCl₂, R¹ = -COCH₂Cl, R²-R⁴ = -H

Synthetisch lassen sich die 1-Trichlormethylheterocyclen durch Pictet-Spengler-Ringschluß aus 3-Indolylethylaminen und Chloral oder durch Bischler-Napieralski-Cyclisierung von 3-Indolylethylamin-trichloracetamiden erhalten.

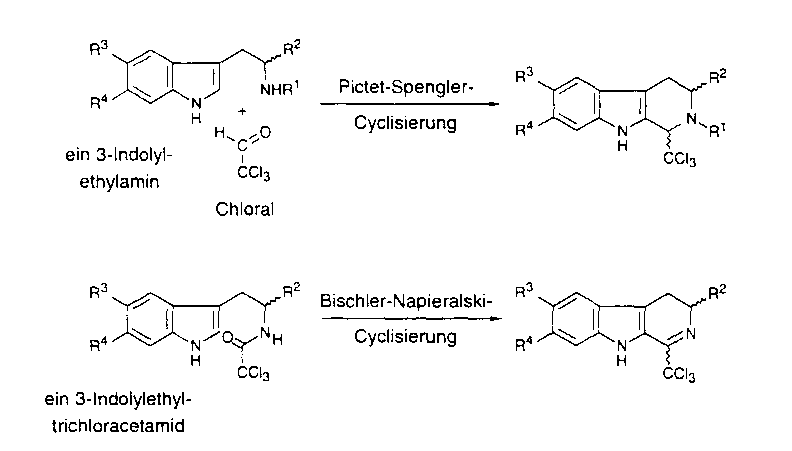

Die so erhaltenen Grundstrukturen können dann durch Reduktion und Oxidation mit geeigneten Reagenzien in die jeweils anderen Grundstrukturen überführt werden. Die Funktionalitäten R¹, R², R³ und R⁴ können durch Verwendung entsprechender Edukte (d.h. entsprechender Amin- bzw. Amidderivate) eingeführt werden. Sie lassen sich ggf. auch nachträglich einbauen oder noch weiter modifizieren. Ganz analog lassen sich auch die Derivate mit Funktionalitäten in 4-, 5- und 8-Position erhalten. β-Carbolinderivate mit anderen Halogenatomen als Chlor lassen sich prinzipiell nach den gleichen Verfahren darstellen.

Es ist damit zu rechnen, daß die Verbindungen mit Grundstruktur (**1**) durch enzymatische und/oder nichtenzymatische Oxidationsreaktionen in die Grundstrukturen (**2**),(**3**) und/oder (**4**),(**5**) biotransformiert werden. Biotransformationsreaktionen sind auch bezüglich der funktionellen Gruppen in Betracht zu ziehen.

Diese neuartigen Chloral-abgeleiteten Heterocyclen zeichnen sich durch das Vorhandensein einer großen, lipophilen Halogen-enthaltenden Gruppe aus. Wahrscheinlich aufgrund dieser lipophilen Eigenschaft ist das Überwinden der Blut-Hirn-Schranke erleichtert.

Die oben als bevorzugt erwähnten N-Alkylverbindungen sind über *N*-Alkylierung herstellbar. So wird z.B. für die Herstellung der alkylierten TaClo-Derivate TaClo-Hydrochlorid in Alkohol, bevorzugt Methanol, gelöst und mit NaHCO₃ und Alkylhalogenid, Alkylsulfat oder Alkylsulfonat umgesetzt. Nach Einengen der Reaktionslösung erfolgt eine Chromatographie, bevorzugt an Kieselgel. Nach Zugabe von einer alkoholischen Mineralsäure-Lösung fällt das *N*-alkylierte Produkt aus. Dieses Verfahren eignet sich natürlich auch zur Darstellung der Verbindungen mit der Grundstruktur (**3**) oder (**5**), sofern Ausgangsverbindungen mit der Grundstruktur (**2**) oder (**4**) eingesetzt werden.

Die N-Alkylverbindungen lassen sich auch durch Reduktion mit Reduktionsmitteln, wie z.B. LiAIH₄ herstellen, dies ist überraschend, da zu erwarten wäre, daß die X-Gruppe, die bevorzugt CCl₃ oder CBr₃ ist, ebenfalls reduziert würde. So läßt sich z.B. ein alkyliertes TaClo-Derivat aus der entsprechenden Acyl-Verbindung und einem Reduktionsmittel herstellen.

Sehr gut wirken auch TaBro-Derivate, die durch Reaktion von einem Tryptamin-Derivat mit Bromal gewonnen werden können. Eine solche Verbindung hat die Formel 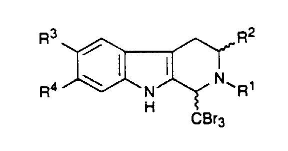 bevorzugt mit:
R¹-R⁴ = -H weiter bevorzugt mit:
R¹ = -CHO, R²-R⁴ = -H
R¹ = -CH₃, R²-R⁴ = -H
R¹ = -C₂H₅, R²-R⁴ = -H
R¹ = -CH₂-C₆H₅, R²-R⁴ = -H
R¹ = -H, R² = -COOCH₃, R³-R⁴ = -H
R¹ = -CH₃, R² = -COOCH₃, R³-R⁴ = -H
R¹ = -C₂H₅, R² = -COOCH₃, R³-R⁴ = -H
R¹ = -CH₂-C₆H₅, R² = -COOCH₃, R³-R⁴ = -H

Die meisten β-Carbolin-Verbindungen fallen bei der Synthese als Racemate an. Diese Enantiomeren-Gemische haben oft pro Gramm Verbindung eine geringere Wirksamkeit als die stereochemisch einheitlichen Wirkstoffe. So geht die Bemühung dahin, die Enantiomere zu trennen. Dies kann durch bekannte Verfahren geschehen oder durch die speziellen Verfahren, die in den nachfolgenden Beispielen angegeben sind.

Eine Verbindung mit der Grundstruktur (**1**), bevorzugt TaClo (R¹, R², R³, R⁴ = H), läßt sich bevorzugt in großen Mengen über den Umweg der /\/-Formyl-Verbindung besonders elegant herstellen. Dabei wird im Falle der Herstellung von TaClo Tryptamin in Ameisensäure mit Chloral umgesetzt. Nach Erwärmen wird ein Alkohol, bevorzugt Methanol, zugegeben. Nach Kristallisation des Produkts (*N*-Formyl-TaClo) wird dieses in verdünnter Mineralsäure, bevorzugt Salzsäure, und Alkohol, bevorzugt Methanol, suspendiert und erhitzt. Nach Einengen der Flüssigkeitsmenge wird der erhaltene Feststoff abfiltriert.

Zur Herstellung der vollaromatischen β-Carbolin-Derivate mit der Grundstruktur (**4**) bzw. (**5**) wird bevorzugt von den Grundstrukturen (**1**),(**2**) bzw. (**3**) ausgegangen und mit Oxidationsmitteln, bevorzugt KMnO₄, oder Dehydrierungskatalysatoren oxidiert.

Die auch bevorzugt verwendeten *N*-Acylverbindungen werden durch Umsetzung der entsprechenden Verbindungen der Grundstrukturen (**1**),(**2**),(3),(**4**) oder (**5**) mit Acylierungsreagenzien, bevorzugt Säurehalogeniden oder -anhydriden umgesetzt, z.B.

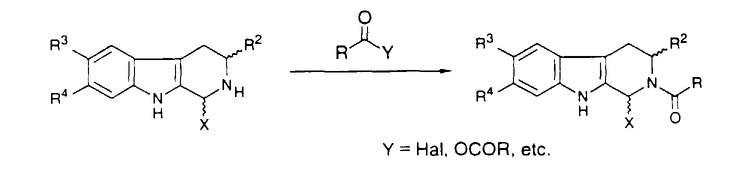

Bei der Synthese von erfindungsgemäßen Verbindungen, bei denen der Rest X eine -CHHal₂-Gruppe sein soll, geht man bevorzugt von der entsprechenden Verbindung mit einer CHal₃-Gruppe aus und reduziert diese mit Reduktionsmitteln, z.B. Natriumborhydrid in absolutem Methanol oder mit Wasserstoff und Pd/C als Katalysator in 80proz. Methanol. Alternativ geht auch eine *de-novo*-Synthese aus z.B. Dihalogenacetaldehyd und den entsprechenden Tryptamin-Derivaten.

Die halogenierten β-Carboline werden als Modellsubstanzen zur Erzeugung neurodegenerativer Erkrankungen eingesetzt. Bei diesen neurodegenerativen Erkrankungen handelt es sich z.B. um Morbus Parkinson oder Morbus Alzheimer. Die Untersuchung neurodegenerativer Prozesse erfolgt dabei z.B. in tierexperimentellen Versuchen sowie mit *in-vitro*-Testsystemen (u.a. in Zellhomogenaten, Neuronenzellkulturen). Ziel ist es, die Ursachen und die Pathogenese neurodegenerativer Erkrankungen aufzuklären.

Bevorzugt ist der Einsatz der halogenierten β-Carboline *in vivo* als langsam wirkende Zellgifte in Langzeit-Tiermodellen, so daß analog der langen präklinischen Phase, wie sie für neurodegenerative Erkrankungen charakteristisch ist, die Ausprägung von Nervenzellverlusten beobachtet werden kann. Dabei wird die Gabe der halogenierten β-Carboline in der Absicht vorgenommen, neurodegenerative Prozesse im Tiermodell hervorzurufen, so daß ein näherer Einblick in die Grundlagen neurodegenerativer Erkrankungen erhalten werden kann. Bevorzugt werden in diesem Zusammenhang Verhaltensänderungen, Störungen bei biochemischen Prozessen sowie morphologische Veränderungen *post mortem* studiert. Für Untersuchungen am Tiermodell werden zum Studium von Verhaltensänderungen bevorzugt Testverfahren eingesetzt, die das spontane Verhalten sowie kognitive und motorische Leistungen der Tiere überprüfen. Zum Studium biochemischer Prozesse *in vivo* werden bevorzugt *in-vivo*-pulsvoltametrische Messungen oder Positronen-Emissionstomographische(PET)-Verfahren verwendet. Soll der Transmitterstoffwechsel im Striatum und in der Substantia nigra pars compacta gemessen werden, erfolgt dies bevorzugt durch *in-vivo*-pulsvoltametrische Messungen. Die Charakterisierung morphologischer Parameter post mortem erfolgt bevorzugt mittels immunhistochemischer Methoden.

Besonders bevorzugt erfolgt der Einsatz halogenierter β-Carboline *in vitro* zur Induktion neurodegenerativer Prozesse in Neuronenzellkultursystemen sowie in weiteren *in-vitro*-Modellen (u.a. in Gewebehomogenaten, angereicherten Zellfraktionen). Ziel ist es, biochemische Mechanismen und morphologische Veränderungen an Neuronen in Zellkultursystemen eingehend zu studieren. Bevorzugt werden hierbei immunhistochemische und biochemische Untersuchungsverfahren eingesetzt.

Weiterhin werden halogenierte β-Carboline und ihre Vorstufen sowohl *in vivo* (im Tiermodell) als auch *in vitro* (in Neuronenzellkulturen und Gewebehomogenaten) zur Aufklärung von Mechanismen, die zur Entstehung, Bioaktivierung, Akkumulation und metabolischer Transformation neuroaktiver Substanzen führen, eingesetzt. Bevorzugt werden im Rahmen dieser Studien autoradiographische Verfahren und chromatographisch-massenspektroskopische Methoden angewendet.

Langfristig ist geplant, aus den Erkenntnissen, die aufgrund des Einsatzes halogenierter β-Carboline im Tiermodell oder Zellkultursystemen zur Induktion neurodegenerativer Prozesse gewonnen wurden, neue mögliche Medikamente zu entwickeln und zu erproben.

Vorteilhaft bei der Verwendung der halogenierten β-Carboline ist, daß die dadurch hervorgerufenen Effekte einen progredienten Verlauf zeigen. Dadurch läßt sich besonders gut analog der langen präklinischen Phase, wie sie für neurodegenerative Erkrankungen charakteristisch sind, die Ausprägung an Nervenzellverlusten beobachten. Die halogenierten β-Carboline sind damit insbesondere geeignete Modellsubstanzen, um Erkenntnisse über die Ursache und das Fortschreiten neurodegenerativer Erkrankungen zu erhalten.

Beispielhaft sei auf die nachfolgenden Beispiele und die darin enthaltenen Formeln der Verbindungen verwiesen.

Die vorliegende Erfindung wird nun weiter mit Bezug auf die anhängenden Figuren beschrieben, von denen
- Fig. 1.: das analytische Verfahren zum Nachweis von halogenierten β-Carbolinen aus biologischer Matrix am Beispiel der Identifizierung von TaClo aus Blutproben von Ratten, die chronisch mit Chloralhydrat (10 mg/kg, *i.p.*) und Tryptaminhydrochlorid (5 mg/kg *i.p.*) behandelt worden waren, zeigt,
- Fig. 2.: die motorische Aktivität von Ratten nach intraperitonealer Applikation von TaClo (0.2 mg/kg oder 0.4 mg/kg) für 20 Tage und 0.4 mg Apomorphin *s.c.,* 1 Stunde nach der letzten TaClo-Injektion zeigt,
- Fig. 3.: die lokomotorische Aktivitäten von Tieren 15 - 17 Tage nach der ersten Injektion von Tryptaminhydrochlorid und Chloralhydrat *i.p.* und 10 - 22 Stunden nach der letzten Injektion zeigt,
- Fig. 4.: den Einfluß einer unilateralen intranigralen Applikation von MPTP (174 µg in 2 µl) auf das voltametrisch gemessene DOPAC-Signal im Striatum der Ratte, gemessen 1 und 3 Wochen nach MPTP-Applikation zeigt,
- Fig. 5.: den Einfluß einer unilateralen intranigralen Applikation von MPP⁺ (30 µg in 2 µl) auf das voltametrisch gemessene DOPAC-Signal im Striatum der Ratte, gemessen 1 und 3 Wochen nach MPP⁺-Applikation zeigt,
- Fig. 6.: den Einfluß einer unilateralen intranigralen Applikation von TaClo (10 µg in 2 µl) auf das voltametrisch gemessene DOPAC-Signal im Striatum der Ratte, gemessen 1 und 3 Wochen nach TaClo-Applikation, zeigt,
- Fig. 7.: den Einfluß einer unilateralen intranigralen Applikation von *N*-Me-TaClo (10 µg in 2 µl) auf das voltametrisch gemessene DOPAC-Signal im Striatum der Ratte, gemessen 1 und 3 Wochen nach *N*-Me-TaClo-Applikation, zeigt,
- Fig. 8.: den Einfluß einer unilateralen intranigralen Applikation von DDH-TaFlu (10 µg in 2 µl) auf das voltametrisch gemessene DOPAC-Signal im Striatum der Ratte, gemessen nach 1 Woche nach DDH-TaFlu-Applikation, zeigt,
- Fig. 9.: den Einfluß einer unilateralen intranigralen Applikation von TaBro (10 mg in 2 ml) auf das voltametrisch gemessene DOPAC-Signal im Striatum der Ratte, gemessen nach 1 Woche nach der TaBro-Applikation, zeigt.

Die vorliegende Erfindung wird nun mit Bezug auf die folgenden Beispiele näher beschrieben.

### Beispiele

### Beispiel 1

Synthetische Zugänge zur Grundstruktur (**1**):
1.1. Die Pictet-Spengler-Kondensation könnte auch bei einer *in-vivo-*Entstehung von 1-Trichlormethyl-β-carbolinderivaten im Säugerorganismus der zentrale Schritt sein: 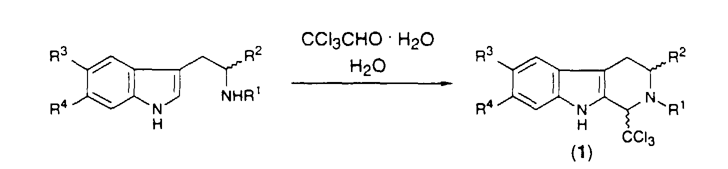
   In 5 ml Wasser werden 100 mg (0.62 mmol) Tryptamin und 205 mg (1.24 mmol) Chloralhydrat gelöst und 32 h bei 40°C gerührt. Das Lösungsmittel wird zur Trockene eingedampft, der Rückstand an Kieselgel (Laufmittel: Petrolether/Chloroform = 1 : 3 v/v) chromatographiert. Die erhaltene Fraktion wird mit methanolischer HCI versetzt, das Hydrochlorid aus Methanol/Ether umkristallisiert, und man erhält 82 mg (0.25 mmol, 40.5%) TaClo (Hydrochlorid) in Form farbloser, pulvriger Kristalle, die sich oberhalb von 230°C zersetzen.
1.2. Für präparative Zwecke in größerem Maßstab eignet sich besser die Umsetzung der Amine mit Chloral in wasserfreien Medien wie z.B. Toluol, Dioxan oder Trifluoressigsäure: 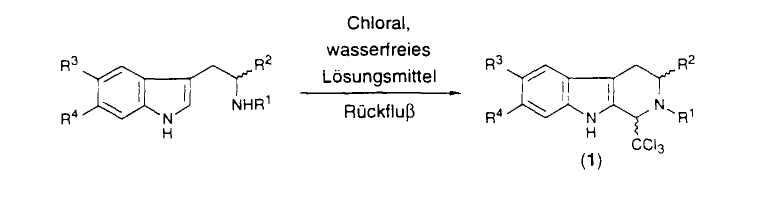 Eine Lösung von 100 mg (0.62 mmol) Tryptamin und 0.5 ml (5.1 mmol) Chloral in 5 ml Dioxan wird 5 min unter Rückfluß erhitzt. Nach Erkalten wird das Lösungsmittel abdestilliert, der Rückstand an Kieselgel (Laufmittel: Dichlormethan) chromatographiert, das Eluat mit methanolischer HCI versetzt und vom Lösungsmittel befreit. Nach Umkristallisieren des Rohproduktes aus Methanol/Ether erhält man 106 mg (0.33 mmol, 52.1%) TaClo (Hydrochlorid) in Form farbloser, pulvriger Kristalle, die sich oberhalb von 230°C zersetzen.
1.3. Eine deutliche Verbesserung ergibt sich durch längeres Erhitzen einer Lösung von Amin und Chloral in Ameisensäure auf 70°C: 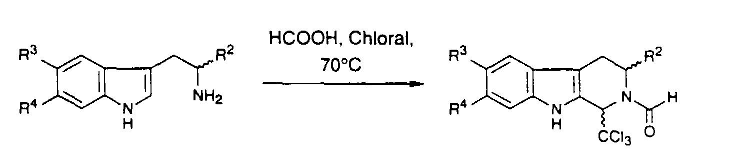
   Diese Reaktion ist besonders gut geeignet für die Darstellung großer Mengen von sehr reinem TaClo: 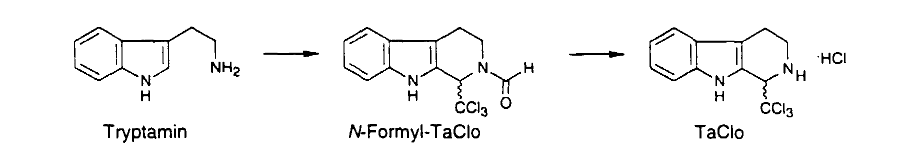
   a) Zu einer Lösung von 100 g (624 mmol) Tryptamin (R² = R³ = R⁴ = H) in 150 ml Ameisensäure werden unter Rühren 91.4 ml (137 g; 936 mmol) Chloral zugetropft. Anschließend wird die Reaktionslösung zwei Stunden auf 70°C erwärmt. Nach dem Abkühlen werden 250 ml Methanol zugegeben. Man läßt zwei Tage bei Raumtemperatur stehen, filtriert dann das ausgefallene Rohprodukt ab und kristallisiert dieses aus Methanol. Man erhält 145 g (73 %) *N*-Formyl-TaClo (R² = R³ = R⁴ = H) als beige Kristalle mit Schmp. 223°C (Zers.).
   b) 18 g *N*-Formyl TaClo werden in 300 ml Methanol und 60 ml 35proz. Salzsäure suspendiert und 2.5 Stunden zum Rückfluß erhitzt. Anschließend wird die Reaktionslösung im Vakuum auf ein Viertel eingeengt und der erhaltene Feststoff abfiltriert. Man erhält 18 g (97 %) TaClo (Hydrochlorid) als farbloses Kristallpulver mit Schmp. 230°C (Zers.).
1.4 Tetrahydro-β-carbolinderivate lassen sich auch durch Reduktion z.B. mit Natriumcyanoborhydrid aus Derivaten mit der Grundstruktur (**2**) bzw. (**3**) erhalten: 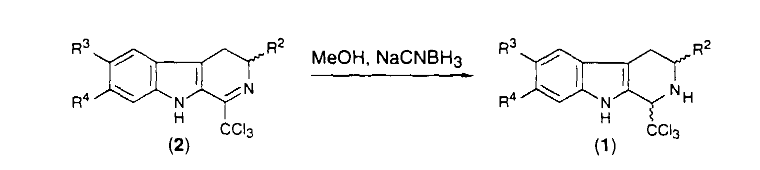
   Zu einer Lösung aus 45 mg (0.16 mmol) 1,2-Didehydro-TaClo in 2 ml einer Mischung aus gleichen Teilen wasserfreiem Chloroform und wasserfreiem Methanol gibt man bei pH 4 und 0-5°C 11 mg (0.18 mmol) Natriumcyanoborhydrid. Nach 2 h quencht man mit etwas Aceton, verdampft das Lösungsmittel und chromatographiert an Kieselgel (Laufmittel: Chloroform). Man versetzt mit methanolischer HCI und erhält nach Kristallisieren aus Methanol/Ether 10.3 mg (0.03 mmol, 20.2%) TaClo (Hydrochlorid), welches sich oberhalb von 230°C zersetzt.

### Beispiel 2

Synthetische Zugänge zur Grundstruktur (**2**) bzw. (**3**):
2.1. Durch Umsetzung des jeweiligen Tryptamin-trichloracetamids mit Phosphorpentoxid in siedendem Xylol (Bischler-Napieralski-Reaktion) erhält man Derivate mit der Grundstruktur (**2**): 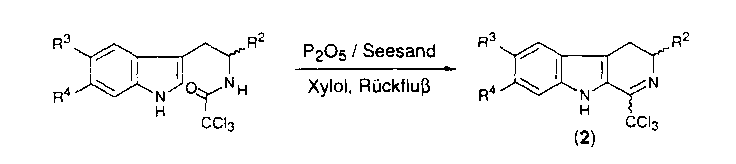
   Zu einer Lösung von 2 g (6.55 mmol) Tryptamin-trichloracetamid in 150 ml wasserfreiem Xylol gibt man bei 110°C in mehreren Portionen ein Gemisch aus 17 g (120 mol) Phosphorpentoxid und 34 g Seesand. Man erhitzt bei starkem Rühren mit einem KPG-Rührwerk 24 h unter Rückfluß. Nach dem Abkühlen filtriert man ab, wäscht mit Petrolether nach und hydrolysiert den Rückstand vorsichtig mit 200 g Eis. Unter Kühlung versetzt man die so erhaltene stark saure Lösung mit festem Natriumhydroxid bis pH 5 und extrahiert mit Essigester. Die vereinigten organischen Phasen werden über Magnesiumsulfat getrocknet, das Lösungsmittel eingedampft und der Rückstand an Kieselgel (Laufmittel: Dichlormethan) chromatographiert. Man erhält nach Kristallisation aus Methanol/Dichlormethan 50 mg (0.17 mmol, 2.6%) 1,2-Didehydro-TaClo in Form hellbeiger Nadeln, die sich oberhalb von 156°C zersetzen.
2.2. Derivate mit der Grundstruktur (**2**) bzw. (**3**) erhält man auch durch Oxidation der 1,2,3,4-Tetrahydro-β-carbolinderivate (Grundstruktur (**1**)) mit diversen Oxidationsmitteln, z.B. mit Kaliumpermanganat in Tetrahydrofuran oder in Aceton: 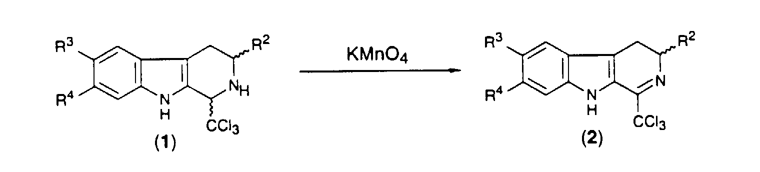
   Ähnlich lassen sich nun auch 1-Trichlormethyl-*N*-alkyldihydro-β-carboliniumsalze **3** gewinnen: 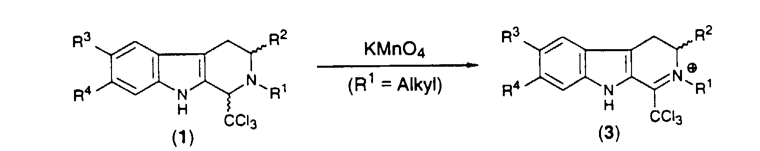 Eine Lösung von 70 mg (0.22 mmol) TaClo (Hydrochlorid) in 15 ml wasserfreiem Tetrahydrofuran wird mit 120 mg (0.76 mmol) Kaliumpermanganat versetzt und 3.5 h bei Raumtemperatur gerührt. Vom Niederschlag wird abfiltriert, das Filtrat zur Trockene eingedampft und an Kieselgel chromatographiert (Laufmittel: Chloroform/Petrolether = 3:2 v/v) Nach Umkristallisation aus Dichlormethan/Ether erhält man 8.2 mg (0.029 mmol, 13.3%) 1,2-Didehydro-TaClo in Form hellbeiger Nadeln, die sich oberhalb von 156°C zersetzen.
2.3. Durch Umsetzung von *N*-Alkylverbindungen der Grundstruktur (**1**) mit Wasserstoffperoxid erhält man das *N*-Oxid, welches dann mit Trifluoressigsäureanhydrid in das 1,2-Didehydroderivat überführt wird:

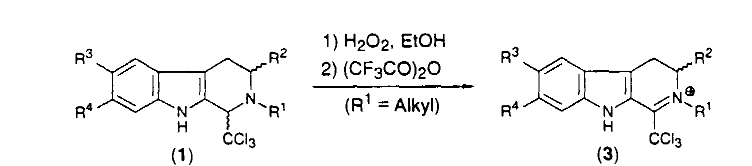

### Beispiel 3

Synthetische Zugänge zur Grundstruktur (**4**) bzw. (**5**):
3.1. Die vollaromatischen 1-Trichlormethyl-β-carboline können durch Oxidation der Derivate mit der Grundstruktur (**1**), (**2**) bzw. (**3**) oder (**4**) bzw. (**5**) mit verschiedenen Oxidationsmitteln (z.B. KMnO₄) oder Dehydrierungskatalysatoren (z.B. Pt) erhalten werden:

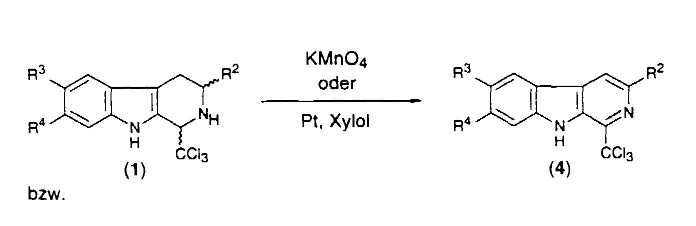

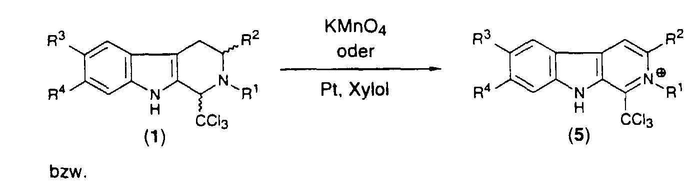

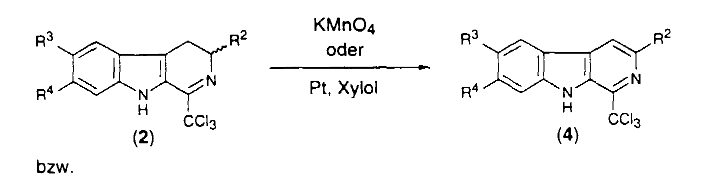

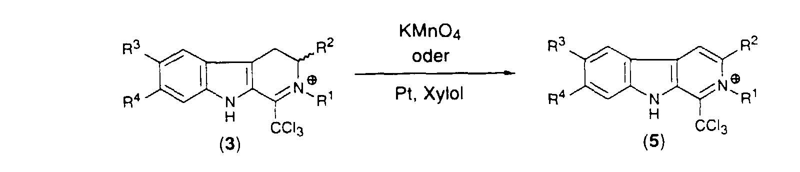

Eine Suspension von 200 mg (0.61 mmol) TaClo (Hydrochlorid) in 25 ml Aceton und 6 ml Chloroform wird auf 50°C erhitzt und mit 320 mg (2.02 mmol) Kaliumpermanganat versetzt. Nach 30 min wird die Reaktionslösung filtriert, vom Lösungsmittel befreit und an Kieselgel (Laufmittel: Methyl-*tert.*-butylether/Petrolether = 1 : 2 v/v) chromatographiert. Das Eluat wird zur Trockene eingeengt. Man erhält 11 mg (0.04 mmol, 6%) 1-Trichlormethyl-β-carbolin als beigen Feststoff, der sich oberhalb von 110°C zersetzt.

### Beispiel 4

### Variation der funktionellen Gruppen

Variation der Restes R¹ :
4.1. Die *N*-Alkylierung gelingt u.a. mit Alkylhalogeniden, -sulfaten oder Alkylsulfonaten (z.B. Trifluormethylsulfonsäurealkylestern) z.B. in Gegenwart von Natriumhydrogencarbonat in Methanol oder in Dimethylsulfoxid: 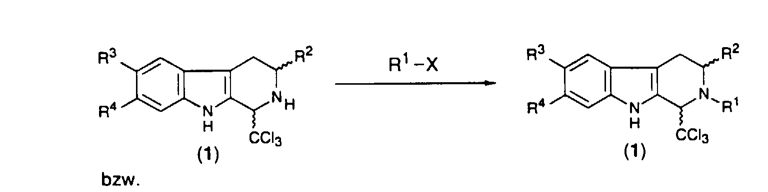 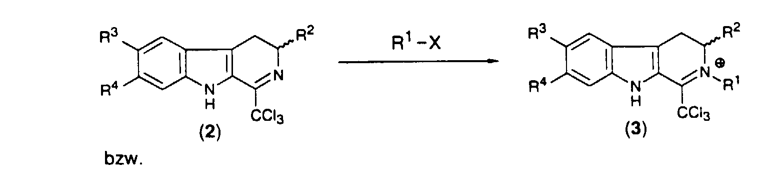 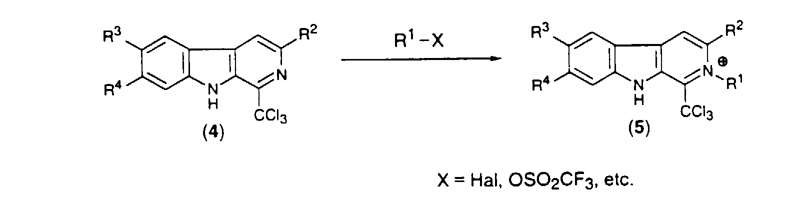
   Zu einer Lösung von 1.0 g (3.07 mmol) TaClo (Hydrochlorid) in 40 ml Methanol werden 1.0 g (11.9 mmol) NaHCO₃ und 2 ml (4.34 g, 30.7 mmol) Methyliodid gegeben. Danach wird 60 Stunden bei Raumtemp. unter Lichtausschluß gerührt. Anschließend wird die Reaktionslösung zur Trockene eingeengt und der Rückstand mit einer Mischung aus Methyl-*tert*.-butylether und Petrolether (1 : 2 v/v) als Fließmittel an Kieselgel chromatographiert. Das Eluat wird mit einem geringen Überschuß methanolischer HCI-Lösung versetzt. Das dabei ausfallende *N*-methylierte TaClo (Hydrochlorid) wird vom Lösungsmittel befreit und im Ölpumpenvakuum getrocknet. Man erhält 0.67 g (1.97 mmol, 64%) der *N*-methylierten Verbindung in Form eines leicht gelblichen amorphen Pulvers welches sich beim Erhitzen auf 96°C zersetzt.
   Trotz Reduktionempfindlichkeit der CCl₃-Gruppe lassen sich *N*-Alkylverbindungen überraschenderweise auch aus den *N*-Acylderivaten mit Reduktionsmitteln wie z.B. Lithiumaluminiumhydrid erhalten: 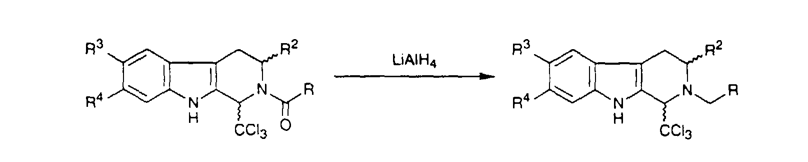
   In einer Lösung von 50 mg (0.16 mmol) von *N*-Formyl-TaClo in 5 ml trockenem Tetrahydrofuran werden 25 mg AlCl₃ suspendiert. Nach 15 min Rühren bei Raumtemp. versetzt man mit einem geringen Überschuß Lithiumaluminiumhydrid und rührt weitere 15 min. Dann wird mit Wasser hydrolysiert und mit 10 ml Ether versetzt. Nach Filtration wird die Lösung bis zur Trockene eingeengt. Der Rückstand wird mit einer Mischung aus Methyl-*tert*.-butylether und Petrolether (1 : 2 v/v) als Fließmittel an Kieselgel chromatographiert. Das Eluat wird mit einem geringen Überschuß an methanolischer HCI-Lösung versetzt und bis zur Trockene eingeengt. Man erhält 31 mg (0.09 mmol, 60%) der *N*-Methyl-Verbindung als Hydrochlorid in Form eines leicht gelblichen amorphen Pulvers welches sich beim Erhitzen auf 96°C zersetzt.
4.2. Die *N*-Acylverbindungen erhält man durch Umsetzung der Derivate mit der Grundstruktur (**1**) mit Acylierungsreagenzien wie z.B. Säurehalogeniden oder -anhydriden: 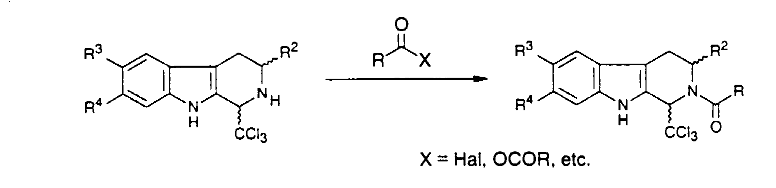
   Zu einer Lösung von 100 mg (0.31 mmol) TaClo (Hydrochlorid) und 170 µl (124 mg, 1.23 mmol) Triethylamin in 10 ml Dichlormethan gibt man bei 0°C eine Lösung von 66 µl (72 mg, 0.92 mmol) Acetylchlorid in 2 ml Dichlormethan. Nach 20 min. Rühren bei Raumtemp. wäscht man zweimal mit je 5 ml 1 M Salzsäure und dann bis zur neutralen Reaktion mit Wasser. Man trocknet die organische Phase über Magnesiumsulfat und engt im Vakuum ein. Nach Umkristallisation aus Methanol erhält man 92 mg (0.28 mmol, 90%) *N*-Acetyl-TaClo als farblose Kristalle, die sich oberhalb von 215°C zersetzen.

Variation des Restes R²:
4.3. Die gewünschten Derivate mit Esterfunktion in 3-Stellung erhält man am einfachsten, wenn die entsprechenden Tryptophanester mit Chloral kondensiert werden. Aber auch eine nachträgliche Umwandlung z.B. durch Veresterung oder sonstige Umwandlung (z.B. Amidierung, etc.) ist möglich: 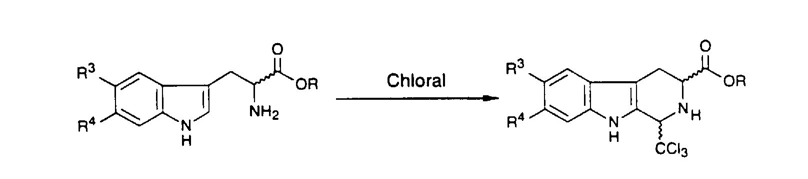
   Eine Lösung aus 530 mg (2.09 mmol) L-Tryptophan-methylester (Hydrochlorid) und 2.44 ml (25.0 mmol) Chloral in 5.3 ml Ameisensäure wird 15 h bei Raumtemp. gerührt. Nach Säulenchromatographie des Eindampfrückstandes an Kieselgel (Laufmittel: Chloroform/Methanol) und Umkristallisieren aus Aceton/Petrolether erhält man 385.5 mg (0.81 mmol) des chromatographisch schneller eluierenden *cis*-Diastereomeren als weiße watteartige Kristalle und 312.3 mg (1.22 mmol) des langsameren *trans*-Diastereomeren in Form nadelartiger Kristalle. Gesamtausbeute 706.8 mg (2.03 mmol, 83.7%).
4.4 Entsprechende Alkohole R² = -CH₂-OH entstehen bei Behandlung der Ester R² = -COOR mit Reduktionsmitteln wie z.B. Natriumborhydrid; wahlweise kann die Reaktion auch so geführt werden, daß zusätzlich die CCl₃-Gruppe reduziert wird (vgl. 5.2). 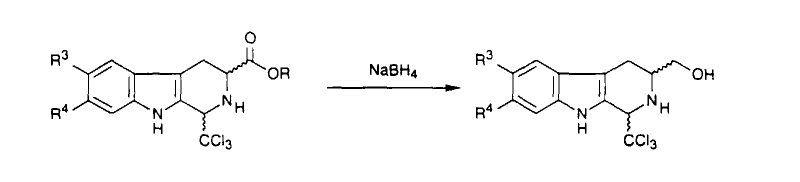
   Eine Lösung von 150 mg (0.43 mmol) des sich von L-Tryptophanmethylester ableitenden (1*R*, 3*S*)-Heterocyclus in 40 ml Methanol wird unter Kühlung und Rühren portionsweise mit 2.3 g (60.1 mmol) Natriumborhydrid versetzt. Die Lösung wird noch 15 min bei Raumtemp. gerührt und dann mit verdünnter Salzsäure auf pH 6-7 gebracht. das Lösungsmittel wird abgedampft, der Rückstand mit Isopropanol digeriert und das Filtrat an Kieselgel (Laufmittel: Chloroform/Methanol = 15 : 1 v/v) chromatographiert. Man erhält dabei 52 mg (0.16 mmol, 37%) des (1*R*, 3*S*)-konfigurierten Alkohols, der sich oberhalb von 123°C zersetzt. [a]_{D}²⁵ = -71.4 (c = 0.5 in Methanol).
   Auch die primäre Alkoholfunktion kann ihrerseits nach Standardverfahren weiter modifiziert werden u.a. durch Oxidation zum Aldehyd, *O*-AIkylierung, *O*-Acylierung, Dehydratisierung, etc.).
4.5 Amide R² = COONR'R" erhält man bei Behandlung der Ester R² = COOR mit einer Lösung des Amins HNR'R" in Methanol (z.B. R = CH₃, R' = R" = H), sowie durch Kondensation entsprechender Tryptophanamide mit Chloral: 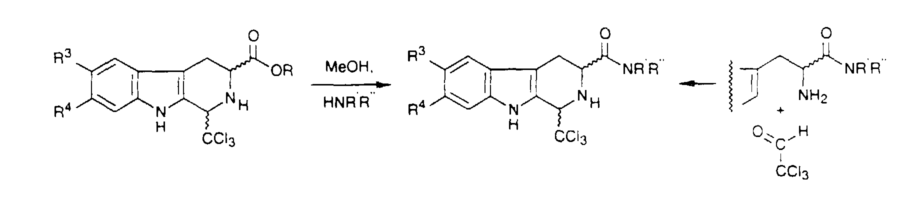
   Auch die Amidfunktion kann durch Standardverfahren weiter verändert werden, u.a. durch Reduktion, Dehydratisierung, *O*- oder *N*-Alkylierung, etc.

Variation der Reste R³ und/oder R⁴:
4.6. In 6- und/oder 7-Stellung substituierte (z.B. alkoxylierte) Derivate lassen sich sehr einfach aus dem entsprechend substituierten Tryptaminderivat und Chloral erhalten (z.B. R³ = OCH₃, R⁴ = H oder R³ = H, R⁴ = OCH₃):

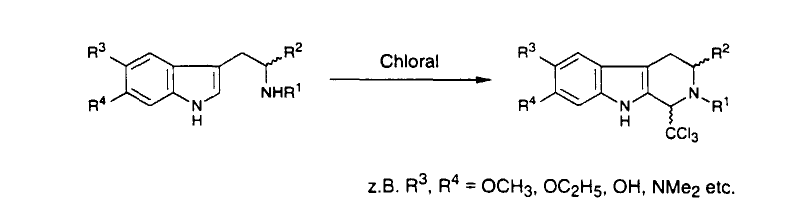

Zu einer Suspension aus 200 mg (0.88 mmol) 5-Methoxy-tryptamin (Hydrochlorid) in 7 ml Toluol gibt man 1.5 ml (2.27 g, 15.4 mmol) Chloral und erhitzt 150 min unter Rückfluß. Anschließend wird die Reaktionslösung bis zur Trockene eingeengt. Nach Chromatographie an Kieselgel (Laufmittel: Chloroform/Methanol = 7 : 1 v/v) wird das erhaltene Öl in das Hydrochlorid überführt. Man erhält nach Umkristallisation aus Methanol/Ether 238 mg (0.67 mmol, 76.4%) 1-Trichlormethyl-6-methoxy-1,2,3,4-tetrahydro-β-carbolin als leicht bräunliche, quaderförmige Kristalle, die sich oberhalb 166°C zersetzen.

Die so erhaltenen Heterocyclen lassen sich durch Standardverfahren weiter verändern, z.B. durch *O*-Alkylierung, *O*-Acylierung, *O*-Dealkylierung, Oxidation, etc. Beispielsweise gelingt so die Darstellung freier Hydroxyverbindungen besonders günstig durch Spaltung der entsprechenden Methylether z.B. mit Lewis-Säuren wie Bortribromid oder mit Protonensäuren: 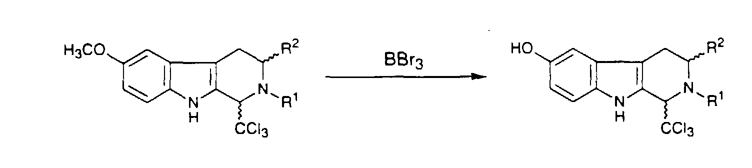 150 mg (0.42 mmol) 6-Methoxy-1,2,3,4-tetrahydro-β-carbolin werden in 10 ml Dichlormethan aufgenommen, auf -65°C abgekühlt und mit 300 µl (792 mg, 3.16 mmol) Bortribromid versetzt. Die Reaktionsmischung läßt man langsam auf Raumtemp. erwärmen und rührt dann bei Raumtemp. bis kein weiterer Umsatz mehr festgestellt werden kann (DC-Kontrolle, Kieselgelplatte, Laufmittel: Chloroform/Methyl-*tert*.-butylether = 3 : 1 v/v). Nach nochmaligem Abkühlen auf 0°C wird durch Zugabe von Wasser überschüssiges Bortribromid hydrolysiert. Die Reaktionsmischung wird nachfolgend dreimal mit Ether extrahiert, und die vereinigten Etherphasen werden mit Magnesiumsulfat getrocknet. Nach Abdestillieren des Lösungsmittels erhält man einen braunen Feststoff, der zur Reinigung an Kieselgel (Laufmittel: Methyl-*tert.*-butylether) chromatographiert wird. Man erhält nach Entfernen des Lösungsmittels 61 mg (0.20 mmol, 47.5%) 1-Trichlormethyl-6-hydroxy-1,2,3,4-tetrahydro-β-carbolin, welches sich beim Erhitzen über 124°C zersetzt.

### Beispiel 5

Variation der Halogen-enthaltenden Gruppe X
5.1 Die Dehydrohalogenierung der 1-Trichlormethylgruppe zur 1-Dichlormethylenfunktion findet sehr leicht unter Baseneinfluß statt z.B. beim Erhitzen mit methanolischer Kaliumhydroxidlösung; alternativ läßt sich die Dichlormethylenstruktur (und ähnliche Partialstrukturen) auch durch Bischler-Napieralski-Synthese aus dem Dichloracetamid gewinnen: 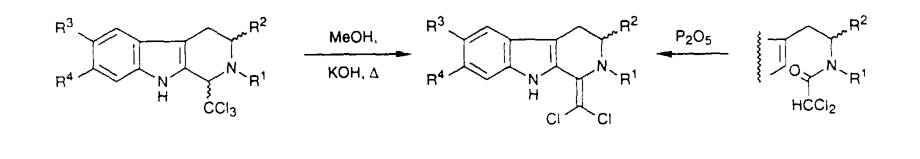
   a) 500 mg (1.53 mmol) TaClo werden in 50 ml Methanol suspendiert und mit einer Lösung von 0.3 g (5.35 mmol) Kaliumhydroxid in 15 ml Methanol versetzt. Nach 5 min. Erhitzen auf Rückflußtemp. wird die gelbe Lösung abgekühlt, mit 3 g Kieselgel versetzt und vom Lösungsmittel befreit. Zur Abtrennung von anorganischen Salzen wird das so erhaltene Pulver auf eine kurze Kieselgelsäule aufgegeben und mit Methyl-*tert*.-butylether/Petrolether = 1 : 2 v/v eluiert. Das Eluat wird mit methanolischer HCI versetzt, vom Lösungsmittel befreit und aus wenig Methanol umkristallisiert. Man erhält 249 mg (0.85 mmol, 56%) Dichlormethylen-TaClo (Hydrochlorid) als orange Kristalle, die sich oberhalb von 180°C zersetzen.
   b) 1 g (3.15 mmol) *N*-Formyl-TaClo werden in 50 ml Methanol suspendiert und mit einer Lösung von 0.6 g (10.70 mmol) Kaliumhydroxid in 30 ml Methanol versetzt. Man erhitzt 10 min auf Rückflußtemperatur und läßt abkühlen. Die erhaltene Lösung wird mit 6 g Kieselgel versetzt und vom Lösungsmittel befreit. Zur Abtrennung von anorganischen Salzen wird das so erhaltene Pulver auf eine kurze Kieselgelsäule aufgegeben und mit Methyl-*tert*.-butylether/Petrolether = 1 : 2 v/v eluiert. Man erhält nach Verdampfen des Lösungsmittels 822 mg (2.92 mmol, 93%) Rohprodukt, welches aus wenig Methanol umkristallisiert wird. Es fallen 491 mg (1.75 mmol, 56%) *N*-Formyl-Dichlormethylen-TaClo als farblose Kristalle aus, die sich oberhalb 187°C zersetzen. Durch Einengen der Mutterlauge läßt sich weiteres Produkt isolieren, welches geringfügig mit der N-deformylierten Verbindung verunreinigt ist.
5.2 Zur Synthese schwächer (z.B. nur zweifach) halogenierter Vertreter läßt sich die CCl₃-Gruppe leicht mit verschiedenen Reduktionsmitteln reduzieren, z.B. mit Natriumborhydrid in absolutem Methanol oder mit Wasserstoff und Pd/C als Katalysator in 80proz. Methanol. Alternativ kann die Substanz auch *de novo,* z.B. aus Dichloracetaldehyd und den entsprechenden Tryptamin-Derivaten gewonnen werden: 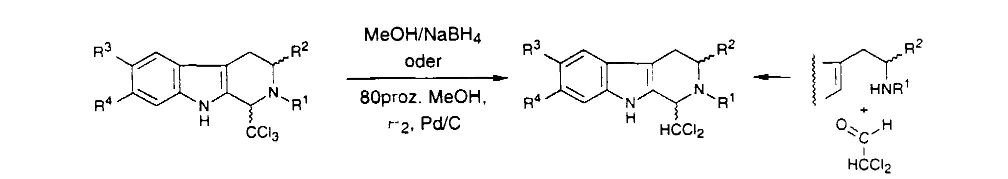
   2.0 g (6.13 mmol) TaClo (Hydrochlorid) werden in 60 ml Methanol gelöst und unter Eiskühlung in mehreren Portionen mit insgesamt 10 g (0.26 mol) Natriumborhydrid versetzt. Es entsteht ein dicker Brei, der mit 30 ml Methanol versetzt wird und noch 4 h bei Raumtemperatur gerührt wird. Nach Zugabe von 10 g Kieselgel wird vom Lösungsmittel befreit, und das erhaltene Pulver wird auf eine Kieselgel-Säule (Laufmittel: Methyl-*tert*.-butylether/Petrolether = 1 : 2 v/v) aufgegeben und chromatographiert. Das Eluat wird mit methanolischer HCI versetzt, vom Lösungsmittel befreit und aus Methanol/Methyl-*tert.*-butylether umkristallisiert. Man erhält 402 mg (1.38 mmol, 22.5%) 1-Dichlormethyl-TaClo (Hydrochlorid).
   Ähnlich können auch Analoga mit längerkettigen Substituenten an C-1 z.B. aus halogenierten Aldehyden gewonnen werden: 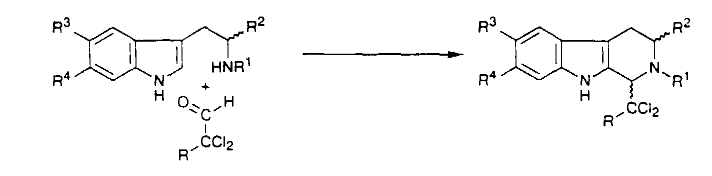
5.3 Zu Analoga mit anderen Halogenen, z.B. den ganz neuen 1-Tribrommethylverbindungen gelangt man durch Pictet-Spengler-Reaktion der Amine mit Bromal in Dioxan, Toluol, Dichlormethan und Aceton. Die Aufarbeitung ist jedoch relativ schwierig, so daß besser die gut kristallisierende *N*-Formylverbindung hergestellt und isoliert wird. Dazu wird das Amin mit Bromal in Ameisensäure bei Raumtemperatur gerührt. Es entstehen dabei zu etwa gleichen Teilen die *N*-Formyl- und die NH-Verbindung. Durch Zugabe von Formylierungsreagenz kann letztere nachformyliert werden, so daß einheitlich *N*-acyliertes Produkt vorliegt. Weitere Umsetzungen sind analog zu den 1-Trichlormethyl-β-carbolinen möglich: 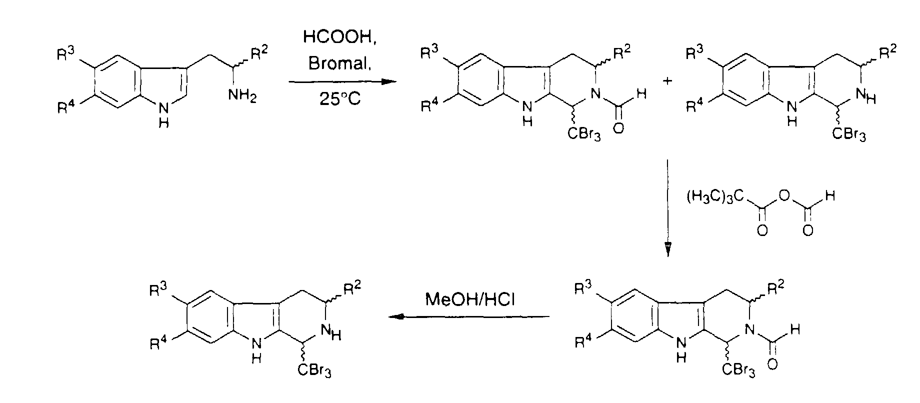
   Weitere Herstellungs- und Umwandlungsmöglichkeiten der CBr₃-Verbindungen folgen den o.g. Verfahren zur Synthese der CCl₃-Analoga.
   5.0 g (30.9 mmol) Tryptamin, 17.4 g (61.8 mmol) Bromal und 7 ml Ameisensäure werden 5 h bei Raumtemp. gerührt und anschließend mit 4.41 g (33.9 mmol) Formylpivaloylanhydrid versetzt. Nach weiteren 6 h Rühren bei Raumtemp. wird die Reaktionslösung mit Eiswasser hydrolysiert, der ausgefallene Feststoff abfiltriert und mit 20 ml Methyl-*tert*.-butylether gewaschen. Man erhält 7.05 g (15.7 mmol, 51%) der *N*-formylierten Verbindung (TaBro) als farbloses Kristallpulver mit Zersetzungspunkt 172°C.
   Zur Deformylierung werden 1.0 g (2.23 mmol) der *N*-formylierten Verbindung in einer Lösung von 2.8 ml konzentrierter Salzsäure in 20 ml Methanol suspendiert und 7 h unter Rückfluß erhitzt. Anschließend wird die Reaktionslösung im Vakuum auf 1/5 eingeengt und der ausgefallene Feststoff abfiltriert. Man erhält 0.92 g (2.00 mmol, 90% d.Th.) TaBro in Form seines Hydrochlorids als farbloses Kristallpulver. Schmp.: 162°C (Zers.).
   Analoge Umsetzungen betreffen auch die chemisch deutlich stabileren Derivate mit einer 1-Trifluormethylgruppe. 1-Trifluormethyl-3,4-didehydro-β-carbolin läßt sich gut nach den bei den 1-Trichlormethylderivaten geschilderten Methoden darstellen, z.B. in einer Bischler-Napieralski-Reaktion (vgl. 2.1): 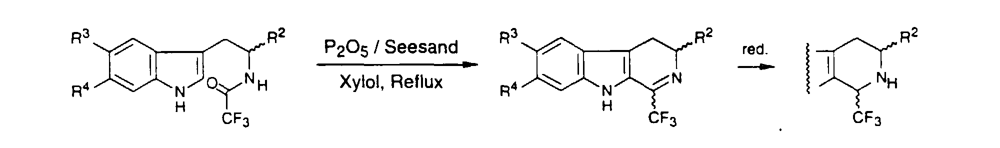 510 mg (2.00 mmol) Trifluoracetyltryptamid werden in 50 ml wasserfreiem Xylol bei 110°C innerhalb von 3 - 45 min in mehreren Portionen mit einem Gemisch aus 5 g Phosphorpentoxid und 10 g Seesand versetzt. Die Dehydratisierung erfolgt bei 6stdg. Erhitzen auf Rückflußtemperatur. Nach Abkühlen auf Raumtemperatur wird abfiltriert und der Rückstand in 200 g Eis/Wasser aufgeschlämmt. Unter Eiskühlung versetzt man die saure Lösung mit NaOH bis pH 8 - 9, extrahiert erschöpfend mit Dichlormethan und trocknet die vereinigten organischen Phasen über Natriumsulfat. Nach Einengen im Vakuum wird das so erhaltene Rohprodukt an Kieselgel (Laufmittel: Ether) chromatographiert. Man erhält 180 mg (0.76 mmol, 38%) leicht gelb gefärbte Kristalle vom Schmp. 84°C.
   Auch hier sind, wie oben für die CCl₃-Verbindungen beschrieben, nachträgliche Derivatisierungs- und Umwandlungsreaktionen möglich.

### Beispiel 6

Synthese von enantiomerenreinem Material

Chirale Strukturen und damit verschiedene Enantiomere treten bei der Grundstruktur (**1**) für R² = H und bei Grundstruktur (**2**) für R² ≠ H auf.

Wegen der bekannten z.T. extrem unterschiedlichen Aktivität von Enantiomeren (Bild/Spiegelbild) in biologischen Systemen (vgl. den Fall Contergan) werden hier Methoden zur Reingewinnung stereochemisch einheitlicher Wirkstoffe vorgestellt:

Darstellung enantiomerenreiner Verbindungen mit der Grundstruktur (**1**), R² = H:
6.1. Enantiomerenreines Material z.B. mit der Grundstruktur (**1**) läßt sich erhalten, indem man die *N*-Formylverbindungen (R¹ = CHO) langsam aus Methanol oder Aceton kristallisiert. Dabei können enantiomerenreine Kristalle erhalten werden, die sich anschließend ohne Verlust an optischer Reinheit *N*-deformylieren lassen. Der freie Stickstoff läßt sich dann wie oben beschrieben derivatisieren; die Zuordnung der absoluten Konfiguration erfolgt über CD-Spektroskopie oder Kristallstrukturanalyse.
   Eine kaltgesättigte Lösung von *N*-Formyl-TaClo in Aceton wird sorgfältig in ein offenes Kristallisiergefäß filtriert und bei Raumtemp. unter Lichtausschluß stehengelassen. Beim Verdunsten des Lösungsmittels wachsen langsam lange Kristallnadeln. Wenn diese etwa einen Zentimeter lang sind und zwischen 20 und 50 mg wiegen, bricht man die Kristallisation ab. Die Überprüfung der Enantiomerenreinheit einzelner Kristalle gelingt mittels HPLC an chiraler Phase (Chiralcel OD, Daicel Chemical Industries, LTD.; Petrolether/Isopropanol = 70 : 30 v/v; 1 ml/min). Das dabei schneller eluierende Enantiomer hat *S*-Konfiguration, das später eluierende ist entsprechend *R*-konfiguriert. Die Drehwerte des *S*- bzw. *R*-Enantiomeren sind [a]_{D}²⁵ = +87.5° (c = 0.481) bzw. [a]_{D}²⁵ = -87.3° (c = 0.393). 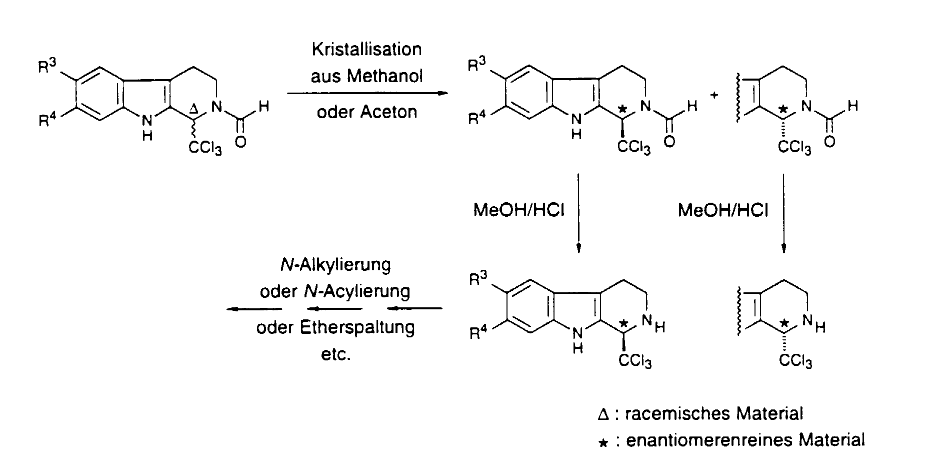
   Nachfolgende Umsetzungen (Deformylierung, *N*-Methylierung usw.) können wie beim racemischen Material beschrieben durchgeführt werden.
6.2 Eine weitere Möglichkeit zur Enantiomerentrennung bietet die Chromatographie an chiraler Phase. Z.B. gelingt die Trennung in die Enantiomerenpaare bei den abgebildeten Substanzen problemlos z.B. durch HPLC mit einer Chiralcel-OD-Phase (Daicel Chemical Industries, LTD.) mit dem Fließmittel Petrolether/Isopropanol (70:30, v/v), aber auch andere Derivate lassen sich auf diese Weise trennen. *N*-Formyl-TaClo, *N*-Me-TaClo und TaClo (R³ = R⁴ = H) lassen sich per HPLC mit den o.g. Bedingungen sehr einfach trennen. Bei diesen drei Verbindungen besitzt jeweils das schneller eluierende Enantiomer *S*- und das langsamer eluierende *R*-Konfiguration: 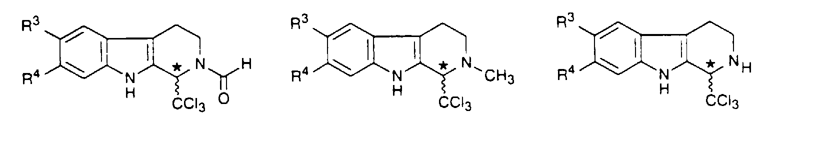
6.3 Auch die Erzeugung von Diastereomeren durch Salzbildung oder durch Derivatisierung am Stickstoff mit chiralen Hilfsreagenzien wie z.B. *R*-1-Phenylethylisocyanat, die nach der Trennung wieder abgespalten werden, bietet einen Zugang zu enantiomerenreinem Material: 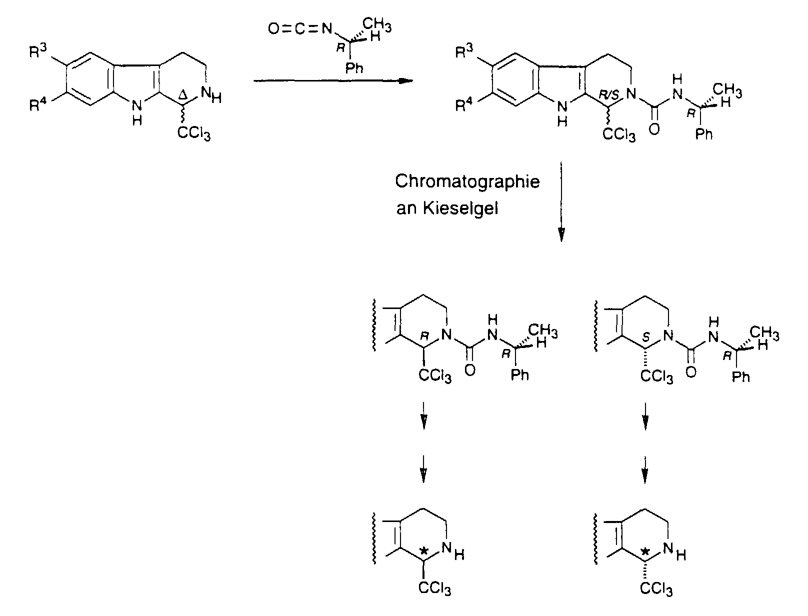
   900 mg (3.11 mmol) TaClo (freie Base) und 4.80 ml (3.42 mmol) (*R*)-α-Phenylethylisocyanat werden zusammen mit katalytischen Mengen 4-Dimethylaminopyridin in 25 ml wasserfreiem Chloroform 4 h unter Rückfluß zum Sieden erhitzt. Nach Entfernen des Lösungsmittels wird der schwarze ölige Rückstand säulenchromatographisch an Kieselgel gereinigt (Laufmittel: Dichlormethan). Man erhält nach Entfernen des Lösungsmittels 886 mg (2.03 mmol, 65%) *N*-[(*R*)-1-Phenylethylcarbamoyl]-1-trichlormethyl-1,2,3,4-tetrahydro-β-carbolin in Form eines weißen Pulvers. Die so erhaltenen Diastereomere lassen sich z.B. durch HPLC oder durch Umkristallisation aus Chloroform/Petrolether oder Dichlormethan/Petrolether trennen. Durch zweimalige Umkristallisation von 100 mg des Diastereomerengemisches enthält man z.B. 31 mg eines reinen Diastereomers in Form farbloser Nadeln, die sich ab 178°C zersetzen.
6.4 Solche gut trennbaren Stereoisomere können auch durch Zugriff auf den "chiral pool", z.B. ausgehend von leicht verfügbaren enantiomerenreinen Tryptophanester-Derivaten erzeugt werden. Die Abspaltung der Esterfunktion liefert dann die enantiomerenreinen Derivate mit der Grundstruktur (**1**). Hier geht man von den kostengünstigen enantiomerenreinen L-Tryptophan-Derivaten aus. So kann die Trennung eines Enantiomerengemisches umgangen werden. Zur Synthese des anderen Enantiomeren bietet sich der Einsatz der ebenfalls kommerziell erhältlichen Derivate aus der D-Reihe an: 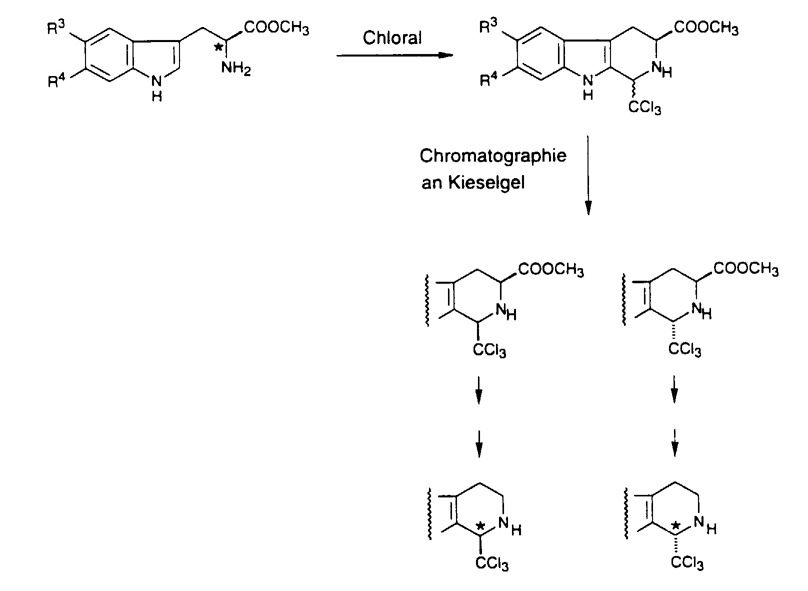
   Darstellung enantiomerenreiner Verbindungen mit der Grundstruktur (**2**) bzw. (**3**), R² ≠ H:
6.5 Die "Hilfschiralität" des Tryptophanderivates kann natürlich auch im Molekül verbleiben und so z.B. zur Synthese enantiomerenreiner 3,4-Dihydro-β-carboline und 3,4-Dihydro-β-carboliniumsalze dienen. Die Abbildung zeigt ein typisches Beispiel:

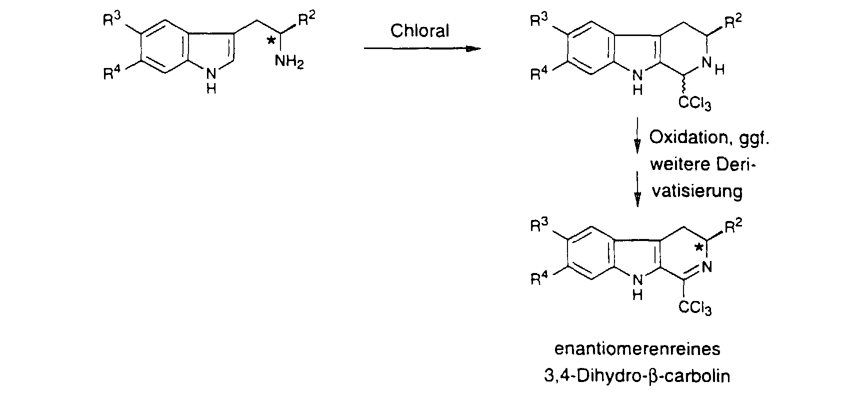

### Beispiel 7

Stereoselektive gezielte Darstellung von diastereomerenreinem Material
Diastereomere treten auf bei der Grundstruktur (**1**) für R² ≠ H.
7.1 Zur Synthese werden die enantiomerenreinen Tryptophanderivate eingesetzt. Man erhält ein Paar enantiomerenreiner Diastereomere, welches sich durch Säulenchromatographie an Kieselgel mit Dichlormethan als Fließmittel trennen läßt (siehe oben).
Ausgehend von enantiomerenreinem *N*_{b}-Benzyltryptophanmethylester erhält man bei der Kondensation mit Chloral in Toluol unter Rückfluß in Gegenwart katalytischer Mengen Trifluoressigsäure in hoher Stereoselektivität ausschließlich die *trans*-Diastereomeren. Nach Abspaltung der Benzylgruppe kann der freie Stickstoff nach den oben beschriebenen Methoden derivatisiert werden:

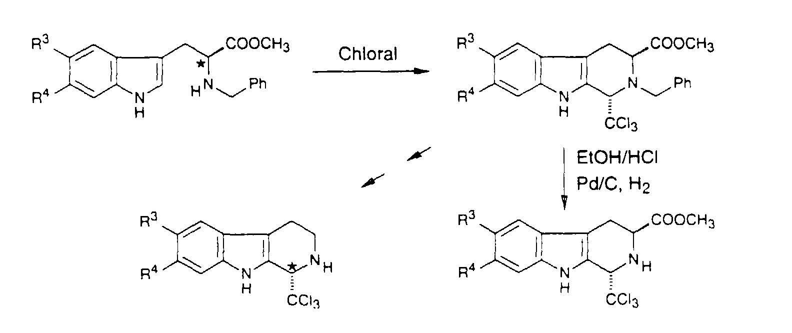

### Beispiel 8

### Nachweis von endogen entstandenen hochhalogenierten β-Carbolinen:

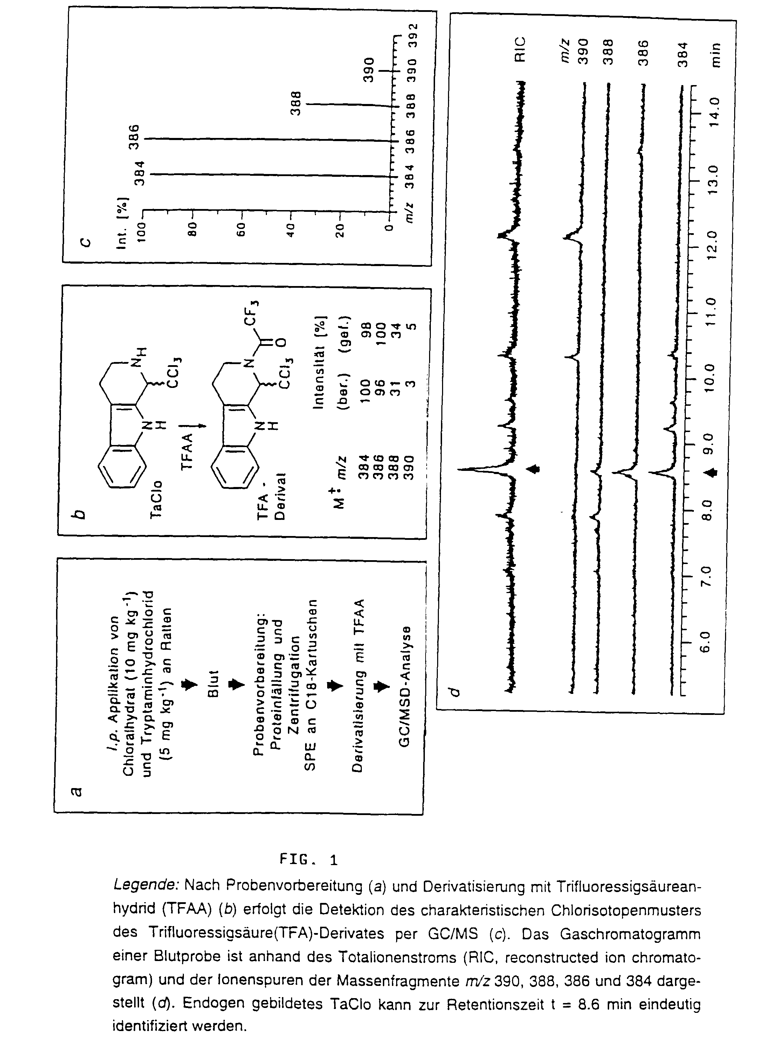

### Am Beispiel von TaClo (vgl. Fig. 1):

Heparinisierte Blutproben (je 1.5 g) von adulten Han-Wistar-Ratten, die 20 Tage lang mit 10 mg/kg Chloralhydrat *i.p.* und 5 mg/kg Tryptamin *i.p.* behandelt worden waren, wurden mit 0.5 ml Acetonitril und 0.5 ml Wasser (alle verwendeten Lösungsmittel hatten HPLC-Qualität) versetzt und für 5 min geschüttelt. Nach Zentrifugation (15 min, 12 000 *g*ₘₐₓ) wurde der Überstand abgenommen und der Rückstand ein weiteres Mal mit 0.5 ml Acetonitril und 0.5 ml Wasser geschüttelt und anschließend zentrifugiert. Die vereinigten Überstände wurden nach Verdünnung mit 4 ml Wasser nochmals 30 min lang zentrifugiert. Der klare Überstand wurde sorgfältig dekantiert und mit 50 µl 0.1 M Natronlauge versetzt. Zur Festphasenextraktion wurden kommerziell erhältliche reversed-phase-Kartuschen (clean-up® C18 octadecyl encapped, 100 mg, United Chemical Technologies, Inc.) mit 2 ml Methanol und anschließend 3 ml Wasser konditioniert. Unter leichtem Vakuum wurde nun die Probenlösung aufgegeben und mit 1 ml Wasser nachgewaschen. Dann wurden die Kartuschen 5 min lang trockengesaugt. Nach Aufgabe von 50 µl einer Methanol / Wasser-Mischung (1:1 v/v) wurde nochmals getrocknet und dann mit 2 ml Methyl-*tert*.-butylether / Chloroform (1:1 v/v) eluiert.

Zur Derivatisierung wurde das Eluat zur Trockene eingedampft und mit 15 mg Natriumhydrogencarbonat, 200 µl Hexan und 20 µl Trifluoressigsäureanhydrid für 20 min bei Raumtemp. gerührt. In einem schwachen Stickstoffstrom wurde nochmals getrocknet, mit 200 µl Toluol extrahiert und anschließend mit 200 µl Wasser gewaschen. Die Toluolphase wurde vorsichtig abgenommen, mit 15 mg Natriumsulfat getrocknet und nach Abdekantieren vom Trockenmittel auf 10 µl eingeengt.

5 µl der so vorbereiteten Probe wurden mit einem HP 5890 Gaschromatographen (Hewlett Packard, Palo Alto, U.S.A.), ausgestattet mit einem On-column-Injektionssystem und einem HP 5971A massenselektiven Detektor, analysiert. Die gaschromatographische Trennung erfolgte auf einer fused-silica Kapillarsäule (HP Ultra 1, 12m x 0.2 mm I.D., Dimethylpolysiloxan, 0.33 um Filmdicke) mit Helium (100 kPa) als Trägergas. Als Temperaturprogramm wählte man 150°C bis 250°C mit einem Gradienten von 5°C / min und hielt noch 5 min bei 250°C. Im SIM-Modus (selective ion monitoring, Elektronenstoßionisation, 70 eV) konnte das charakteristische Molekülion-lsotopenmuster des TaClo-Trifluoressigsäurederivates beobachtet werden (vgl. Fig. 1*c* und 1*e*).

### Beispiel 9

### Einsatz als Modellsubstanzen bei Verhaltensstudien am Tiermodell, z.B. der Ratte:

Eine durch Neurotoxine verursachte Denervation des postsynaptischen Rezeptors im dopaminergen System führt zu einer Rezeptorüberempfindlichkeit. Dadurch wird die Reaktion gegenüber verabreichten DA-Agonisten (z.B. Apomorphin) größer. Zur Überprüfung einer z.B. TaClo-bedingten Überempfindlichkeit dopaminerger Rezeptoren wird der Apomorphin-Test nach Ungerstedt (Ungerstedt et al., 1971) benutzt. Dieser läßt nach *s.c.* Injektion von 0.4 oder 2 mg/kg Apomorphin bei einseitiger Läsion des dopaminergen Systems im Versuchstier in den für die Entstehung des Morbus Parkinson ausschlaggebenden Basalganglien contralaterale Drehungen erkennen und nach mehr oder weniger beidseitiger Schädigung dopaminerger Strukturen bei geringem Schädigungsgrad vermehrte, bei hohem Schädigungsgrad verminderte Lokomotion im Vergleich zum Kontrollkollektiv sichtbar werden.

### Typischer Versuch mit TaClo bei adulten Han-Wistar-Ratten:

Die Verhaltensbeobachtungen fanden während und nach einstündiger Adaption in Beobachtungskäfigen statt. Die Aufzeichnung einzelner spezifischer Parameter wie gelaufene Wegstrecke [DT], Zeit ambulatorischer Bewegungen [AT], Anzahl stereotyper Bewegungen [BMS] und Zeiten stereotyper Bewegungen erfolgte automatisch.

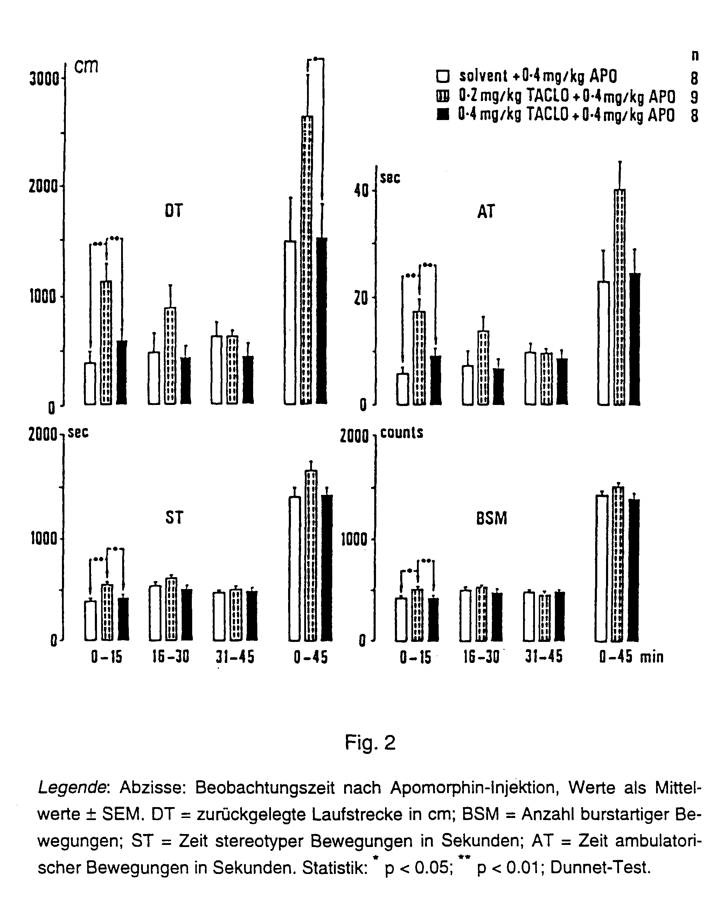

Ratten, die vier Wochen lang TaClo (Hydrochlorid) *i.p.* verabreicht bekommen hatten, zeigten nach Abschluß der Behandlung nach der kleineren Dosis (0.2 mg/kg) eine höhere Empfindlichkeit gegen Apomorphin als diejenigen Tiere, die die größere Dosis (0.4 mg/kg) erhalten hatten. Die lokomotorische Aktivität stieg nach der kleineren Dosierung signifikant an, was auf einen funktionell hypersensitiven postsynaptischen Dopaminrezeptor schließen läßt (vergl. Fig. 2). Die höhere Dosis (0.4 mg/kg) könnte über die schon bei der geringeren Dosis zu beobachtende toxische Wirkung hinaus noch einen weitergehenden Effekt haben: Unter der Einwirkung der höheren Dosis kann ein Dendritenschwund und Verlust synaptischer Verbindungen festgestellt werden, was eine andere neurotoxische Ausgangslage ergibt, auf die der Organismus nach dem Verlust neuronaler Elemente in anderer Weise reagiert.

### Typischer Versuch mit den TaClo-Vorstufen Chloral und Tryptamin bei adulten Han-Wistar-Ratten:

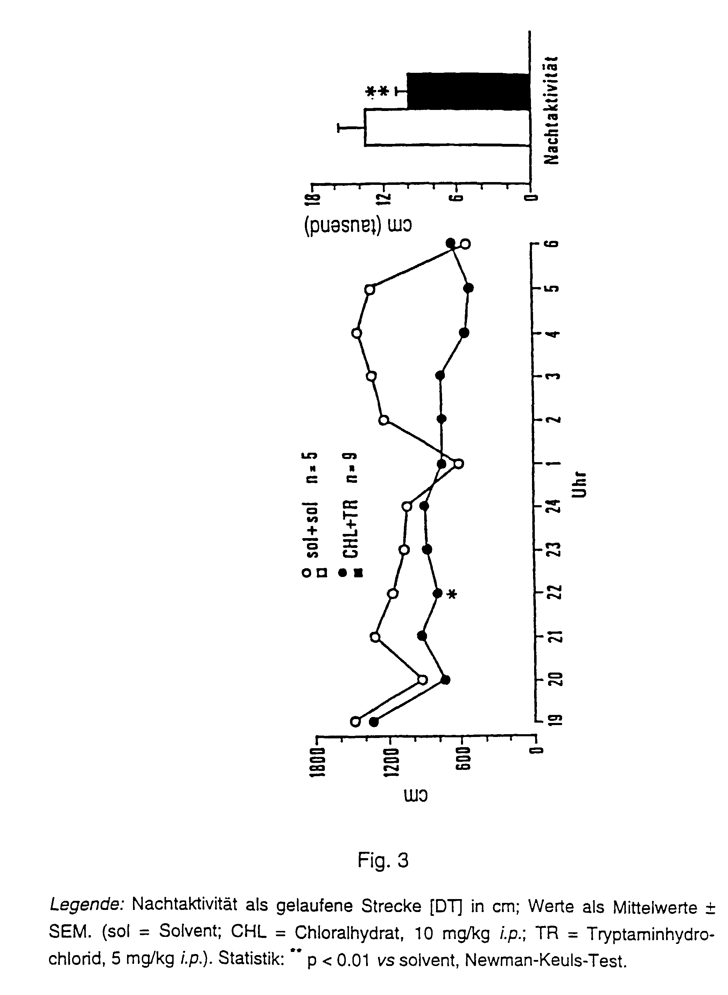

Nach 15- bis 17-tägiger Behandlung mit 10 mg/kg Chloralhydrat und 5 mg/kg Tryptamin-Hydrochlorid *i.p.* zeigt das Rattenkollektiv von n = 9 Ratten in der einstündigen Explorationsphase in einem neuen Umfeld die gleiche mittlere Laufaktivität wie die mit NaCl behandelten Kontrollen (n = 6). Bewertet man jedoch die Tiere aufgrund ihrer Laufaktivität in der Adaptionsphase einzeln, so lassen sich zwei Gruppen bilden, wobei die eine Gruppe in ihrer Laufaktivität praktisch doppelt so aktiv ist wie die andere Gruppe. Bei den passiven Tieren zeigt sich eine signifikante Steigerung der Lauf- und ambulatorischen Aktivität, sowie der stereotypen Aktivität mit vermehrter Putz- und Schnüffelaktivität (p < 0.01 - p < 0.005). Fig. 3 und Tabelle 1 zeigen, daß die Spontaneität und der ultradiane Rhythmus lokomotorischer Aktivität von Tieren nach dieser 15- bis 17-tägigen Kombinationsbehandlung mit Chloralhydrat und Tryptamin nicht ausgebildet ist - ein Zeichen fehlender dopaminerger Aktivität, wie es bei nicht geschädigten Ratten mittels Pulsvoltametrie am DOPAC-Signal gezeigt werden kann (O'Neill und Fillenz, 1985). Dieser Versuch zeigt, daß bei subchronischer Behandlung mit Chloralhydrat und Tryptamin endogen entstandenes TaClo (welches sich in Blut- und Gehirnproben dieser Tiere mit gaschromatographisch-massenspektrometrischen Methoden identifizieren und charakterisieren ließ) aufgrund seiner Auswirkungen auf das dopaminerge System der Ratte 10 und 22 Stunden nach der letzten Injektion der Vorstufen zu einer Beeinträchtigung der Nachtaktivität führt.

**Tabelle 1:**

| | | |
|---|---|---|
| Registrierung der spontanen Nachtaktivitäten im Autotrack-System. Nachtaktivitäten von Tieren 15-17 Tage nach der ersten Injektion von Tryptaminhydrochlorid und Chloralhydrat (5 mg/kg bzw. 10 mg/kg) *i.p.* und 10 - 22 Stunden nach der letzten Injektion. Die Werte als Mittelwert ± SEM während 12 Stunden Beobachtungszeit; Statistik: * p < 0.05 vs solvent; Dunnet-Test. DT = zurückgelegte Laufstrecke in Metern; BSM = counts für burstartige Bewegungen; ST = Zeit stereotyper Bewegungen in Sekunden; AT = Zeit ambulatorischer Bewegungen in Sekunden. | | |

| | Spontane Nachtaktivität | |
|---|---|---|
| | solvent (n = 5) | Chloral + Tryptamin (n = 9) |
| DT[m] | 868.22 ± 146.23 | 618.11 ± 71.40 |
| BMS [c] | 11213.20 ± 879.18 | 8926.89* ± 506.63 |
| ST[s] | 8513.58 ± 605.00 | 6881.16* ± 319.40 |
| AT[s] | 202.38 ± 33.62 | 143.12 ± 16.12 |

### Beispiel 10

### Einsatz als Modellsubstanzen bei in-vivo-pulsvoltametrischen Messungen am Tiermodell:

Es erfolgte die Überprüfung einer neurotoxischen Wirkung der halogenierten β-Carboline. Als Vergleich wurden MPTP bzw. MPP⁺ verwendet, die bei der Maus bzw. bei Primaten zu einer Schädigung dopaminerger Neurone der Substantia nigra führen und damit eine dem Morbus Parkinson ähnliche Symptomatik auslösen. An der Ratte ist es schwierig, ein solches Modell zu etablieren, jedoch führte auch hier die intranigrale Applikation von sowohl MPTP als auch MPP⁺ zu einer Zerstörung dopaminerger Neurone in diesem Areal, verbunden mit entsprechenden motorischen Defiziten.

### Neurotoxizitätsprüfung:

Die 1-Trichlormethyl-β-carboline TaClo und *N*-Me-TaClo wurden im Vergleich zu den bekannten Neurotoxinen MPTP und MPP⁺, wie folgt, auf ihre neurotoxische Wirkung überprüft. Die zu untersuchenden Substanzen (TaClo, *N*-Me-TaClo, DDH-TaFlu, TaBro, MPTP und MPP⁺) wurden unter Narkose mit einer Mikroglaskapillare stereotaktisch nach Koordinaten (A: 4,0 mm; L: 1,9 mm; V: 2,0 mm; ab Interaurallinie) von Paxinos und Watson (1982) unilateral in die rechte Substantia nigra pars compacta der Ratte (Han-Wistar, Zentralinstitut für Versuchstierzucht, Hannover, 250-300 g) injiziert. Appliziert wurde ein Volumen von 2 µl über einen Zeitraum von 15 Minuten.

| Substanz | Dosierung | Lösungsmittel |
|---|---|---|
| 1) MPTP | 174 µg / 2 µl | Physiol. NaCl-Lösung |
| 2) MPP⁺ I⁻ | 30 µg / 2 µl | Physiol. NaCl + Nal |
| | | |
| 3) TaClo | 10 µg / 2 µl | Physiol. NaCl-Lösung |
| 4) N-Me-TaClo | 10 µg / 2µl | Physiol. NaCl-Lösung |

### Validierung des Modells mit den bekannten Neurotoxinen MPTP bzw. MPP⁺:

Im Striatum, dem Projektionsareal der nigralen dopaminergen Neurone, wurde 1 und/oder 3 Wochen nach Applikation der Substanzen der Transmitterstoffwechsel untersucht. Die Untersuchung des Dopaminstoffwechsels wurde dabei in beiden Striata, dem linken Striatum, Projektionsgebiet der unbehandelten linken Substantia nigra, und dem rechten Striatum, Projektionsgebiet der behandelten rechten Substantia nigra, durchgeführt. Die Messung des Dopaminstoffwechsels erfolgte mit Hilfe der *in vivo* differentiellen Pulsvoltametrie (DPV) mit Mikrokohlefaserelektroden, wie sie 1980 von Gonon beschrieben wurde (Gonon *et al.,* 1980). Dabei werden die extrazellulären Spiegel an 3,4-Dihydroxyphenylessigsäure (DOPAC) erfaßt, einem Metaboliten des Transmitters Dopamin. Die Messung wurde am narkotisierten Tier durchgeführt. Die stereotaktische Implantation von 2 Elektroden (rechtes und linkes Striatum) erfolgte nach Koordinaten (A: 8,5 mm; L: ± 3,5 mm; V: 4,5 mm; ab Interaurallinie) von Paxinos und Watson (Paxinos und Watson, 1982). Die voltametrischen Messungen wurden im Abstand von 8 Minuten durchgeführt. 40 Minuten nach Beginn der Messungen applizierte man den Tieren L-DOPA (100 mg/kg *i.p.),* um die Restitutionsfähigkeit des Dopaminstoffwechsels durch dieses Anti-Parkinsonmittel zu überprüfen.

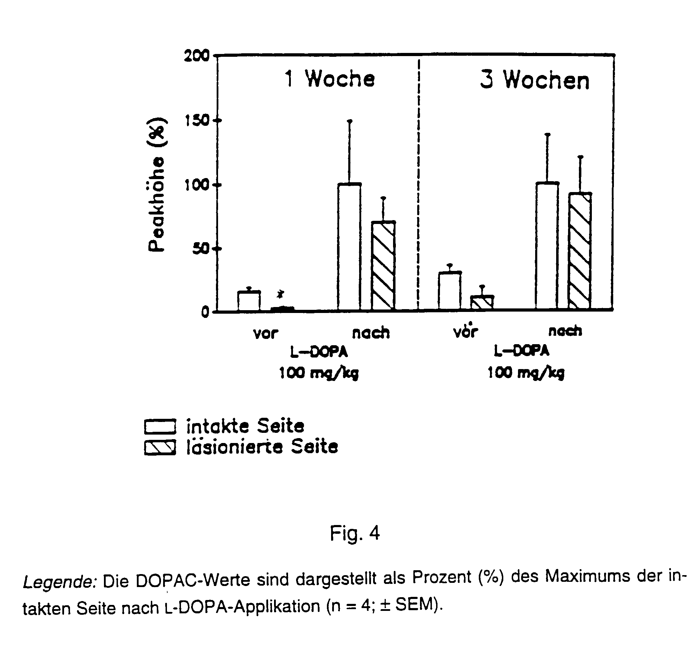

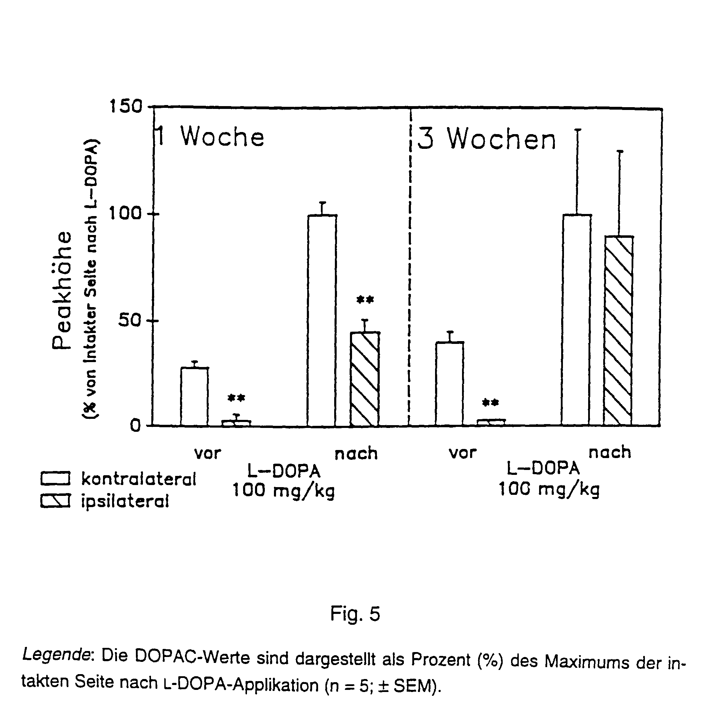

In den Figuren 4 und 5 sind die Wirkungen der intranigralen Applikation von MPTP (Fig. 4) und MPP⁺ (Fig. 5) auf die extrazellulären DOPAC-Konzentrationen vor und nach L-DOPA-Gabe dargestellt. Die Untersuchungen belegen, daß das verringerte DOPAC-Signal auf der injizierten Seite kausal zu einer Verhaltensänderung führte, die mit einem Verlust dopaminerger Neurone verbunden war.

Während der DOPAC-Wert nach einer Woche auf der intakten Seite vor L-DOPA etwa 20% erreichte (Fig. 4), waren auf der läsionierten Seite nur etwa 6% zu messen, was eine signifikante (p < 0.05) Erniedrigung des DOPAC-Signals bedeutet. 3 Wochen nach MPTP-Applikation war der Effekt zwar noch ausgeprägt, jedoch statistisch nicht mehr signifikant. Die Applikation von L-DOPA ergab in beiden Messungen, daß das dopaminerge System den Neuronenverlust durch Gabe dieser Vorstufe kompensieren konnte, was den Beobachtungen bei Parkinsonpatienten entspricht.

Die Fig. 5 verdeutlicht, daß die Wirkung von MPP⁺ wesentlich stärker ausgeprägt war als nach MPTP. Dies wurde sichtbar zum einen durch die hochsignifikante (p < 0.01) langanhaltende Verringerung des DOPAC-Signals auf der injizierten Seite und zum ändern durch die mangelnde Restitutionsfähigkeit des dopaminergen Systems durch DOPA eine Woche nach MPP⁺-Läsionierung.

Anhand dieses MPTP/MPP⁺-Modells wurden die 1-Trichlormethyl-β-carboline, exemplarisch TaClo und *N*-Me-TaClo, auf ihre neurotoxische Wirkung überprüft.

### Typischer Versuch mit TaClo:

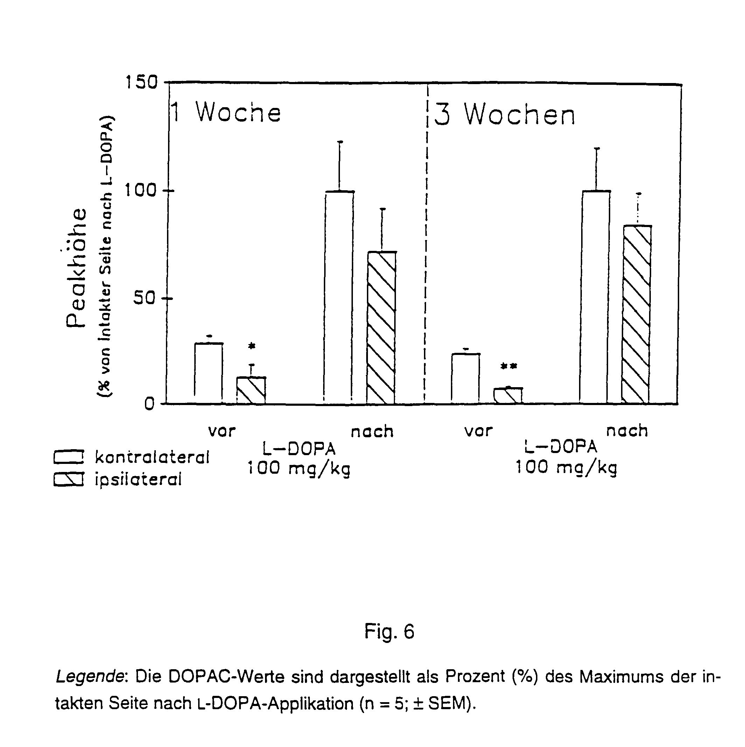

Die einmalige TaClo-Applikation (10 µg in 2 µl) in die rechte Substantia nigra führte dabei zu einer deutlichen Verringerung des DOPAC-Signals auf der ipsilateralen Seite (Fig. 6). Die voltametrischen Messungen in beiden Striata, 1 und 3 Wochen nach TaClo-Applikation, ergaben eine signifikant geringere DOPAC-Konzentration im ipsilateralen Striatum vor L-DOPA-Gabe, 1 Woche: p < 0.05; 3 Wochen: p < 0.01. Diese Untersuchungen belegen eine ausgeprägte Läsion des nigrostriatalen dopaminergen Systems und damit eine neurotoxische Wirkung für diese neue Substanzklasse, exemplarisch für TaClo.

### Typischer Versuch mit N-Me-TaClo:

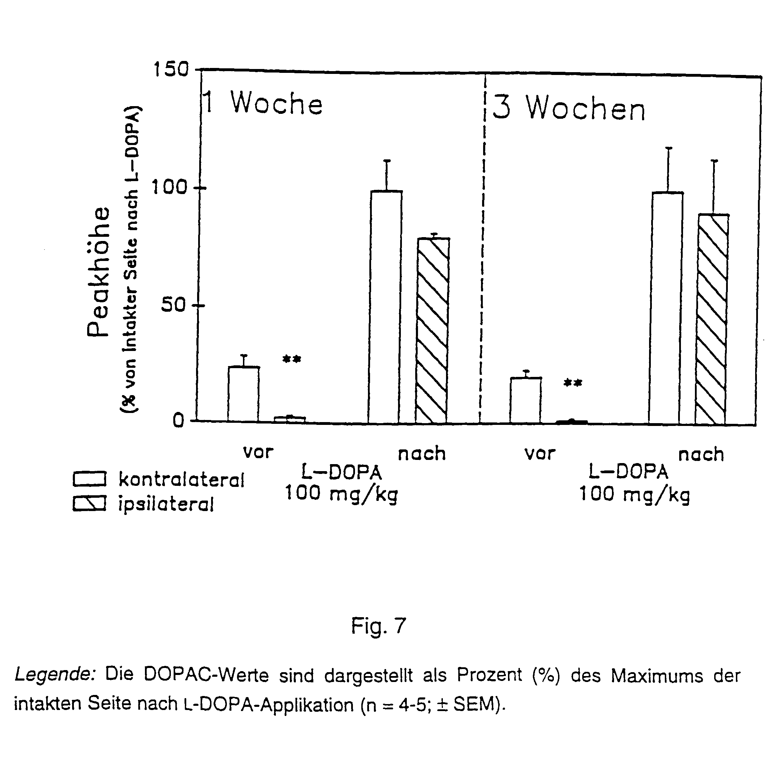

Vergleichbare Befunde wurden für mögliche Metabolite von TaClo, exemplarisch für *N*-Me-TaClo, erhoben. Wieder reduzierte die einmalige unilaterale intranigrale Applikation von *N*-Me-TaClo das DOPAC-Signal im ipsilateralen Striatum signifikant, (p < 0.01 nach einer Woche; p < 0.01 nach drei Wochen) und zwar sogar deutlicher als für TaClo selbst (Fig. 7).

### Typischer Versuch mit DDH-TaFlu und TaBro:

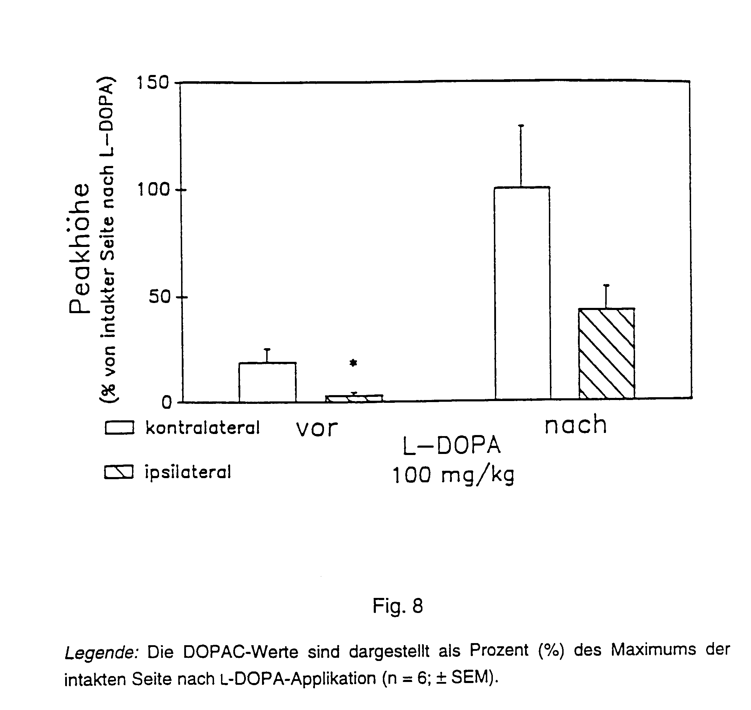

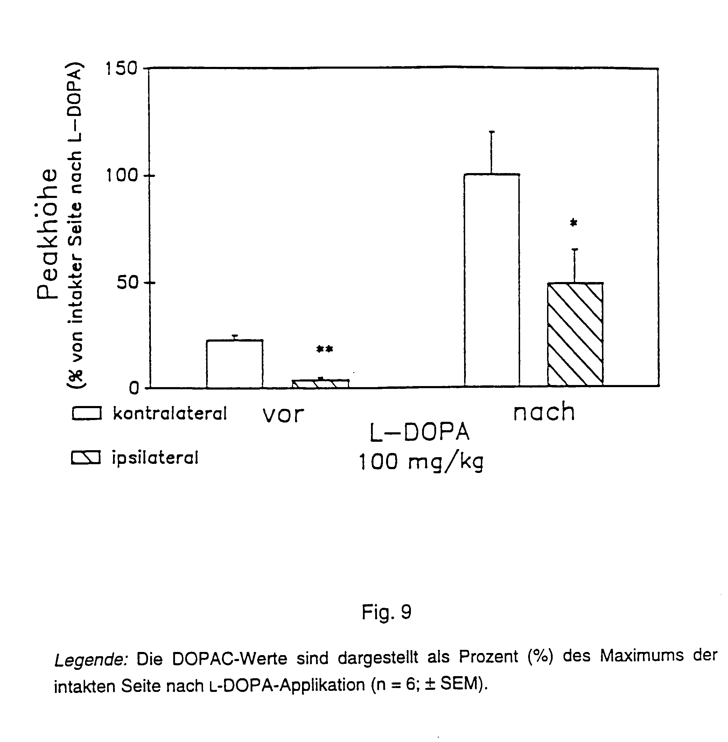

Wie bereits für die Chloral-abgeleiteten Tetrahydro-β-carboline TaClo und *N*-Me-TaClo demonstriert, belegen Untersuchungen mit dem Fluoralabgeleiteten Didehydro-β-carbolin DDH-TaFlu bzw. mit dem Bromalabgeleiteten Tetrahydro-β-carbolin TaBro, daß diese Substanzen Prozesse auslösen, die zu ausgeprägten Läsionen des nigrostriatalen dopaminergen Systems führen. Dies wird offensichtlich aufgrund voltametrischer Befunde für DDH-TaFlu (Fig. 8) und TaBro (Fig. 9), die eine stärkere Reduktion des DOPAC-Signals (für TaFlu: p < 0.05; für TaBro: p < 0.01) zeigen. Damit wird die neurotoxische Wirkung hochfluorierter bzw. bromierter β-Carboline belegt, exemplarisch für DDH-TaFlu und TaBro.

TaClo, *N*-Me-TaClo, DDH-TaFlu und TaBro führen also zu einer Neurodegeneration. Quantitativ betrachtet, sind TaClo und die erwähnten Derivate in ihrer neurotoxischen Potenz wesentlich höher als MPTP einzuschätzen. Die Effekte von *N*-Me-TaClo, DDH-TaFlu sowie TaBro sind vergleichbar oder sogar stärker als die des MPTP-Metaboliten, MPP⁺.

### Beispiel 11

### Einsatz als Modellsubstanzen zu histologischen Studien am Beispiel der Neuronenzellgruppe der Substantia nigra:

### Typischer Versuch mit TaClo:

Die ersten Untersuchungen von drei mit TaClo behandelten Ratten mit quantitativer Auswertung der Substantia nigra pars compacta zeigen, daß TaClo, in einer Konzentration von 0.03 µmol einseitig in die Substantia nigra injiziert, bereits nach einer Woche zu einer eindeutigen Reduktion der Neuronenzahl von 12% auf der läsionierten Seite führt. 6-OHDA, in einer annähernd doppelt so hohen Konzentration von 0.05 µmol injiziert, bewirkt einen 51% Abfall der Neuronenzahl.

Nach Anfärbung der Tyrosinhydroxylase mittels Antikörpern zur Darstellung dopaminerger Neurone zeichnen sich pathologische Veränderungen ab, die nach Behandlung mit TaClo auf der läsionierten Seite zu 3 - 6% nekrotischem Gewebsanteil am Gesamtvolumen führen. Bei 6-OHDA beträgt dieser Anteil 25%.

Zusammenfassend zeigen die Untersuchungen, daß TaClo innerhalb von nur einer Woche Zellausfälle in der Substantia nigra von Ratten verursachen kann. Sie erreichen aber in der gewählten Konzentration noch nicht das Ausmaß der Läsion mit dem klassischen Neurotoxin 6-OHDA, das in diesem Versuchsaufbau zusätzlich in einer höheren Konzentration verabreicht wurde. Die Verabreichung der sehr geringen Konzentration von 0.03 µmol TaClo sollte der Bindung bzw. dem Vorkommen im Gehirn des Menschen bei chronischer Einnahme von Chloralhydrat oder Inkorporation von Trichlorethylen nahekommen. Beim Morbus Parkinson wird z.B. von einer ca. 10- bis 15-jährigen Vorlaufphase, der sogenannten präklinischen Phase, ausgegangen, bei der sich die Nervenzellverluste langsam ausprägen (Riederer und Wuketich, 1976). Dies könnte somit auch eher für ein langsam (schleichend) wirkendes Zelltoxin wie TaClo als für 6-OHDA zutreffen.

### Beispiel 12

### Einsatz als Modellsubstanzen in Primärzellkultur:

Es wurde der Nachweis erbracht, daß 1-Trichlormethyl-β-carboline leistungsfähige neuartige Modellsubstanzen für das Studium neurodegenerativer Prozesse darstellen. Repräsentative Substanzen (z.B. TaClo, *N*-Me-TaClo) dieser Verbindungsklasse zeigen in unterschiedlichen Neurotoxizitätstests neurotoxisches Potential:

In der Zellkultur zeigen 1-Trichlormethyl-β-carboline neurotoxische Eigenschaften, die mit denen von MPP⁺ bzw. MPTP, den bisher bestuntersuchten dopaminergen Neurotoxinen, vergleichbar sind. Das neurotoxische Potential der chlorierten β-Carboline wurde daher mit Methoden untersucht, die sich beim Studium der Wirkung von MPTP und seiner Analoga bewährt haben (Koutsilieri et al., 1993; Michel et al., 1990; Sanchez-Ramos et al., 1988) Deutliche degenerative Veränderungen sind *in vitro* im Akutversuch bei einer Einwirkungszeit von 24 Stunden ab einer Konzentration von 10 - 100 µM feststellbar. Die Substanzen führen zum Zelltod dopaminerger Primärzellen aus dem Mesencephalon - Zellen, die den bei Morbus Parkinson betroffenen Neuronen des Menschen entsprechen. Dem Zelltod dieser immunhistochemisch eindeutig charakterisierten Neuronen (Antikörper auf Tyrosinhydroxylase) gehen morphologische Veränderungen der Zellen voraus. Dabei nimmt die Gesamt-Zelloberfläche ab, die Dendriten und Axone, (zuerst die sekundären und dann die primären Zellfortsätze) gehen verloren. Diese histologischen Befunde (vgl. auch Tabelle 2) wurden durch bildgebende Verfahren erhärtet und quantifiziert. Die 1-Trichlormethyl-β-carboline zeigen auch toxische Eigenschaften in Gliazellkulturen, wie durch histologische Befunde nachgewiesen werden konnte. Für die Gruppe der 1-Trichlormethyl-β-carboline läßt sich eine Beeinflussung der synaptischen Transmitterkonzentration erwarten, wie an Versuchen mit der Grundstruktur TaClo nachgewiesen werden konnte, deren Zusatz konzentrationsabhängig (IC₅₀ 50 µM) zu einer Freisetzung von Dopamin aus den Zellkulturen führt. Dies wurde durch Dopaminbestimmung in den Kulturen mit Hilfe der Hochdruckflüssigkeitschromatographie und elektrochemischer Detektion nachgewiesen. Der Einsatz von ³H-markiertem Dopamin für Aufnahmestudien weist eine Interaktion der 1-Trichlormethyl-β-carboline mit den Dopaminaufnahmestellen in Zellkulturen nach, die eine wichtige Voraussetzung für die Aufnahme dieser Substanzen in das dopaminerge Neuron und die Entfaltung der neurotoxischen Wirkung darstellen (IC₅₀ für TaClo 100 µM) (vgl. Tabelle 2).

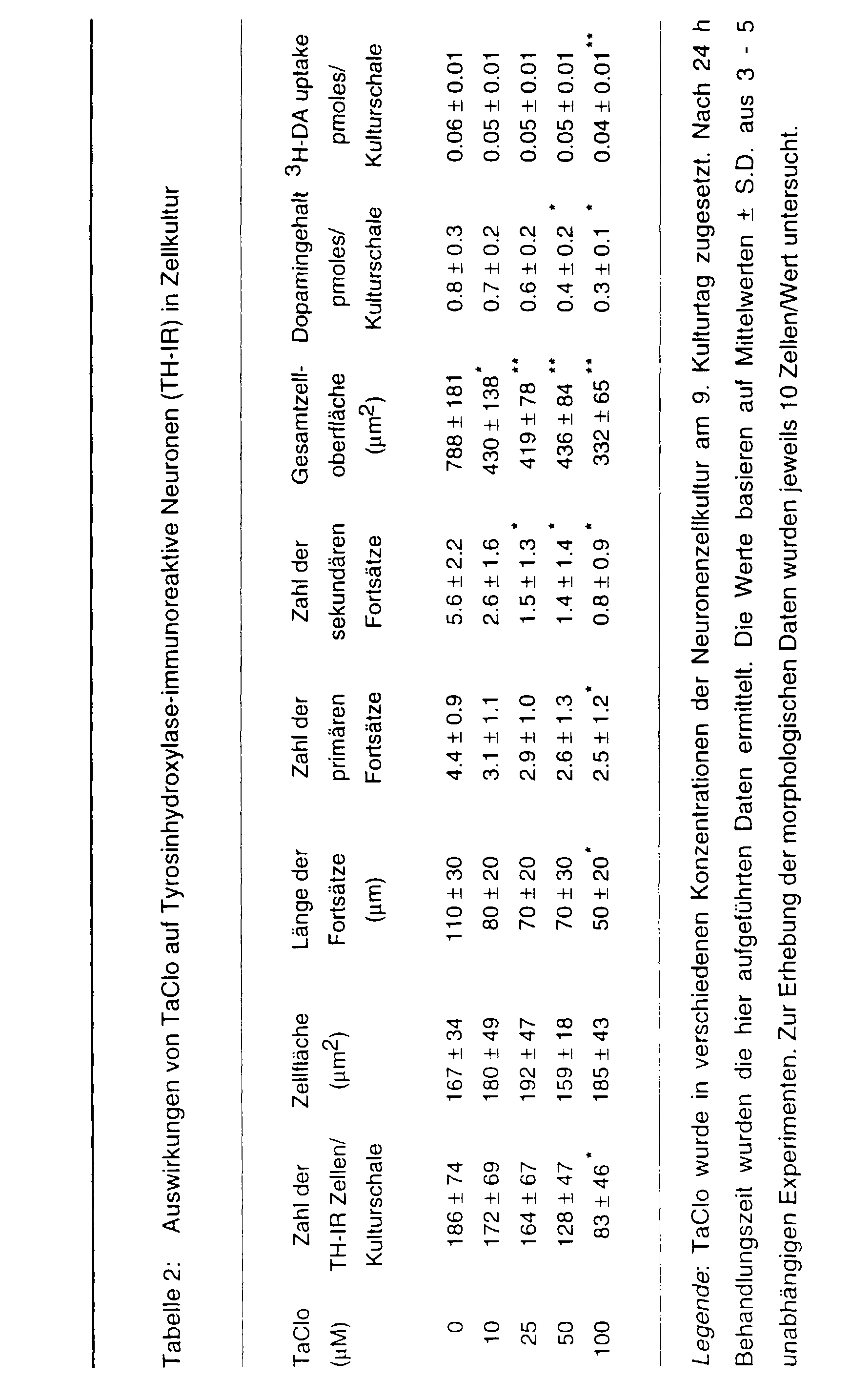

### Literatur

Gonon F, Buda M, Cespuglio R, Jouvet M, Pujol JF. (1980) In vivo electrochemical detection of catechols in the neostriatum of anaesthetized rats: dopamine or DOPAC? *Nature* **286**: 902-904.

Koutsilieri E, Chan WW, Reinitzer D, Rausch WD (1993) Functional changes in cocultures of mesencephalic and striatal neurons from embryonic C57/Bl6 mice due to low concentrations of 1-Methyl-4-Phenylpyridinium (MPP⁺). *J. Neural Transm.* [Gen Sect] **94**:189-197.

Langston JW, Ballard PA, Tetrud JW, Irwin I (1983) Chronic Parkinsonism in humans due to a product of meperidine-analog synthesis. *Science* **219**:979-980.

Michel PP, Dandapani BK, Knusel B, Sanchez-Ramos JR, Hefti F, (1990) Toxicity of 1-methyl-4-phenylpyridinium for rat dopaminergic neurons in culture: selectivity and irreversability. *J. Neurochem.* **54**:1102-1109.

O'Neill RD, Fillenz M (1985) Simultaneous monitoring of dopamine release in rat frontal cortex, nucleus accumbens und striatum: effekt of drugs, circadian changes and correlations with motor activity. *Neuroscience* **16**:49-55.

Paxinos G, Watson C (1982) The rat brain in stereotaxic coordinates. (Academic Press, Sidney).

Riederer P, Wuketich X (1976) Time course of nigro-striatal degeneration in Parkinson's Disease. *J. Neural Transm.* **39**:277-301.

Sanchez-Ramos JR, Michel P, Weiner WJ, Hefti F (1988) Selective destruction of cultured dopaminergic neurons from fetal rat mesencephalon by 1-methyl-4-phenylpyridinium: cytochemical and morphological evidence. *J. Neurochem.* **50**:1934-1944.

Tanner CM (1989) The role of environmental toxins in the etiology of Parkinson's Disease. *Trends Neurosci.* **12**:49-54.

Ungerstedt et al. (1971) Postsynaptic supersensitivity after 6-hydroxydopamine-induced degeneration of nigrostriatal dopamine system. *Acta phyiol. Scand.* [Suppl] **367**:69-93.

## Patentansprüche

1. Verwendung von β-Carbolinen mit der Formel (**I**) 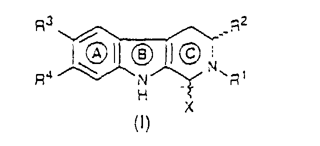 wobei in Ring C eine, zwei oder drei Doppelbindungen vorhanden sind,
X die Bedeutung -C(Hal)₃, -CH(Hal)₂, -CH₂(Hal) oder =C(Hal)₂ mit Hal = F, Cl, Br, I hat, und
die Substituenten R¹, R², R³ und R⁴ dabei folgende Bedeutungen haben:
R¹ : -H, eine C₁-C₁₈-Alkylgruppe (z.B. Methyl, Ethyl, Propyl, *n*-Butyl, *iso*-Butyl, *tert*.-Butyl usw.), -CH₂-CH=CH₂, -CH₂-C₆H₅, eine Acyl- oder Aroylgruppe, mit der Maßgabe, daß R¹ auch ein freies Elektronenpaar sein kann, wenn der Stickstoff an der Ausbildung einer Doppelbindung beteiligt ist,
R² : -H, -COOH, -COOCH₃, -COOC₂H₅, -COOCH₂-CH=CH₂, -COOCH₂-C₆H₅, -COONH₂, -CO₂NR₂, -CH₂OAc, -CH₂-OH, etc.,
R³ : H-, HO-, CH₃O-, C₂H₅O-, C₆H₅-CH₂O-, CH₃COO-, C₂H₅COO-, etc.
R⁴ : H-, HO-, CH₃O-, C₂H₅O-, C₆H₅-CH₂O-, CH₃COO-, C₂H₅COO-, etc.
in vivo im Tiermodell oder in vitro als Modellsubstanzen zur progredienten Erzeugung neurodegenerativer Erkrankungen.

2. Verwendung nach Anspruch 1, wobei es sich bei den neurodegenerativen Erkrankungen um Morbus Parkinson oder Alzheimer'sche Krankheit handelt.

3. Verwendung nach Anspruch 1 oder 2, wobei das β-Carbolin eine der folgenden Formeln (**1**)-(**5**) erfüllt: 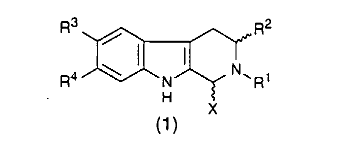 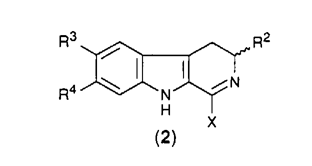 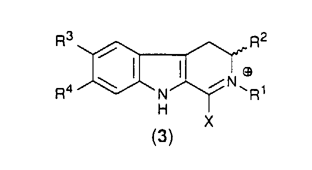 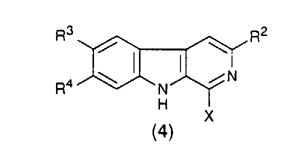 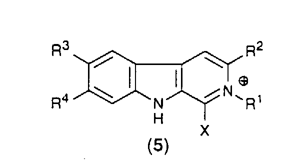 und X, R¹-R⁴ die in Anspruch 1 definierten Bedeutungen haben.

4. Verwendung nach einem der Ansprüche 1-3, wobei in Formel (**I**) R¹-R⁴ = H und X = C(Cl)₃ oder C(Br)₃ oder C(F)₃ ist.

5. Verwendung nach einem der Ansprüche 1-3, wobei in Formel (**I**) R¹ = C₁-C₁₈-Alkyl ist und R²-R⁴ die in Anspruch 1 definierten Bedeutungen haben.

6. Verwendung nach einem der Ansprüche 1-3, wobei R² = -COOH, -COOCH₃, -COOC₂H₅, -COOCH₂-CH=CH₂, -COOCH₂-C₆H₅, -COONH₂, -CO₂NR₂, -CH₂OAc, -CH₂OH ist und X, R¹, R³, R⁴ die in Anspruch 1 definierten Bedeutungen haben.

7. Verwendung nach einem der Ansprüche 1-3, wobei R⁴ = H und R³ = H, HO- CH₃O-, C₂H₅O-, C₆H₅-CH₂O-, CH₃COO-, C₂H₅COO- ist und X, R¹, R² die in Anspruch 1 definierten Bedeutungen haben.

8. Verwendung nach einem der Ansprüche 1-3, wobei R³ = H und R⁴ = H, HO- CH₃O-, C₂H₅O-, C₆H₅-CH₂O-, CH₃COO-, C₂H₅COO- ist und X, R¹-R² die in Anspruch 1 definierten Bedeutungen haben.

9. Verwendung nach einem der Ansprüche 1-3, wobei X eine =C(Hal)₂-Gruppe mit Hal = F, Cl, Br, list und R¹-R⁴ die in Anspruch 1 definierten Bedeutungen haben.

10. Verwendung halogenierter β-Carboline nach Anspruch 3, wobei in Formel (1) die X-Gruppe eine -C(Cl)₃-Gruppe oder eine -C(Br)₃-Gruppe oder eine -C(F)₃-Gruppe ist und R¹-R⁴ die in Anspruch 1 definierte Bedeutung haben.

11. Verwendung halognierter β-Carboline in enantiomerenreiner Form nach Anspruch 3, wobei in Formel (1) die X-Gruppe ein -C(Hal)₃-Gruppe mit Hal = F, Cl, Br, List und R² = H und R¹, R³, R⁴ die in Anspruch 1 definierte Bedeutung haben.

12. Verwendung halogenierter β-Carboline in diastereomerenreiner Form nach Anspruch 3, wobei in Formel (**1**) - (**3**) R² = -COOH, -COOCH₃, -COOC₂H₅, -COOCH₂-CH=CH₂, -COOCH₂-C₆H₅, -COONH₂, -CO₂NR₂, -CH₂OAc, -CH₂OH ist und X, R¹, R³, R⁴ die in Anspruch 1 definierten Bedeutungen haben.

13. Verwendung halogenierter β-Carboline nach Anspruch 3, wobei der Stickstoff im Indolring alkyliert oder acyliert ist und X, R¹-R⁴ die in Anspruch 1 definierten Bedeutungen haben.

14. Verwendung halogenierter β-Carboline nach Anspruch 3, wobei in Formel (**1**) R¹ = -COCCl₃ bzw. -COCH₂Cl und -COCH₂Cl ist und X, R²-R⁴ die in Anspruch 1 definierten Bedeutungen haben.

15. Verwendung halogenierter β-Carboline nach einem der Ansprüche 1-14 mit OH-, OCH₃-, CH₃-, F-, Cl-, Br- und I-Substituenten in den Positionen 4 und/oder 5 und/oder 8 des β-Carbolingerüstes, wobei X, R¹-R⁴ die in Anspruch 1 defininierten Bedeutungen haben.

16. Verwendung halogenierter β-Carboline nach einem der Ansprüche 1-15, wobei die β-Carboline in tierexperimentellen Versuchen zur Untersuchung der Ursachen und Pathogenese neurodegenerativer Erkrankungen sowie zum Studium von Verhaltensveränderungen eingesetzt werden.

## Claims

1. Use of β-carbolines having formula (I) 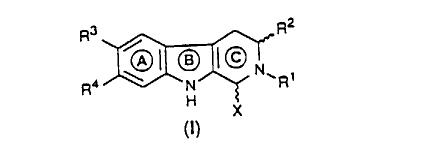 wherein one, two or three double bonds are present in ring C,
X is -C(Hal)3, -CH(Hal)2, -CH2(Hal) or =C(Hal)2 in which Hal = F, Cl, Br, I, and
the substituents R1, R2, R3 and R4 are as defined below:
R1: -H, a C1-C18 alkyl group (e.g. methyl, ethyl, propyl, n-butyl, iso-butyl, tert.-butyl, etc.), -CH2-CH=CH2, -CH2-C6H5, an acyl or aroyl group with the proviso that R1 can also be a free electron pair when the nitrogen is involved in the development of a double bond,
R2: -H, -COOH, -COOCH3, -COOC2H5, -COOCH2-CH=CH2, -COOCH2-C6H5,-COONH2, -C02NR2, CH2OAc, -CH2-OH, etc.,
R3: H-, HO-, CH3O-, C2H5O-, C6H5-CH2O-, CH3COO-, C2H5COO-, etc.
R4: H-, HO-, CH30-, C2H50-, C6H5-CH20-, CH3COO-, C2H5COO-, etc.
in vivo in an animal models or in vitro as model substances for the progressive production of neurodegenerative disorders.

2. Use according to claim 1, wherein the neurodegenerative disorders are parkinsonism or Alzheimer's disease.

3. Use according to claim 1 or 2, wherein the β-carboline meets one of the following formulae (1) to (5): 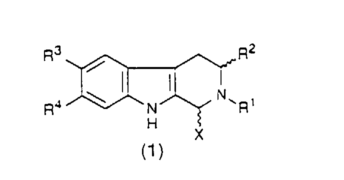 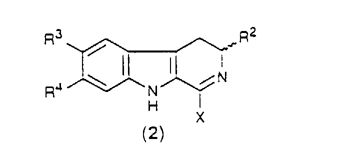 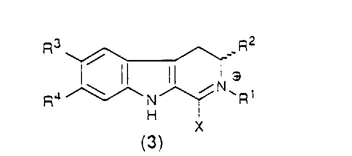 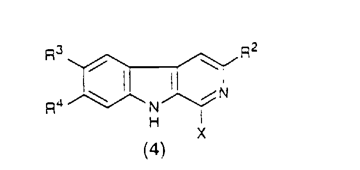 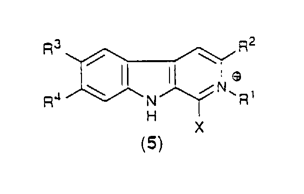 and X, R1 to R4 are as defined in claim 1.

4. Use according to any one of claims 1 to 3, wherein in formula (I) R1 to R4 = H and X = C(Cl)3 or C(Br)3 or C(F)3.

5. Use according to any one of claims 1 to 3, wherein in formula (I) R1 = C1-C18 alkyl and R2 to R4 are as defined in claim 1.

6. Use according to any one of claims 1 to 3, wherein R2 =-COOH, -COOCH3, -COOC2H5, -COOCH2-CH=CH2, -COOCH2-C6H5, -COONH2,-CO2NR2, -CH2OAc, -CH20H and X, R1, R3, R4 are as defined in claim 1.

7. Use according to any one of claims 1 to 3, wherein R4 = H and R3 = H, HO- CH30-, C2H50-, C6H5-CH20-, CH3COO-, C2H5COO- and X, R1, R2 are as defined in claim 1.

8. Use according to any one of claims 1 to 3, wherein R3 = H and R4 = H, HO-, CH30-, C2H50-, C6H5-CH20-, CH3COO-, C2H5COO- and X, R1 to R2 are as defined in claim 1.

9. Use according to any one of claims 1 to 3, wherein X is a =C(Hal)2 group in which Hal = F, Cl, Br, I and R1 to R4 are as defined in claim 1.

10. Use of halogenated β-carbolines according to claim 3, wherein in formula (1) the X group is a -C(Cl)3 group or a -C(Br)3 group or a -C(F)3 group and R1 to R4 are as defined in claim 1.

11. Use of halogenated β-carbolines in enantiomerically pure form according to claim 3, wherein in formula (1) the X group is a-C(Hal)3 group in which Hal = F, Cl, Br, I and R2 = H and R1, R3, R4 are as defined in claim 1.

12. Use of halogenated β-carbolines in diastereomerically pure form according to claim 3, wherein in formulae (1) to (3) R2 =-COOH, -COOCH3, -COOC2H5, -COOCH2-CH=CH2, -COOCH2-C6H5, -COONH2,-C02NR2, -CH20Ac, -CH20H and X, R1, R3, R4 are as defined in claim 1.

13. Use of halogenated β-carbolines according to claim 3, wherein the nitrogen in the indole ring is alkylated or acylated and X, R1 to R4 are as defined in claim 1.

14. Use of halogenated β-carbolines according to claim 3, wherein in formula (1) R1 = -COCCl3 and -COCH2Cl, respectively, and -COCH2Cl and X, R2 to R4 are as defined in claim 1.

15. Use of halogenated β-carbolines according to any one of claims 1 to 14 with OH, OCH3, CH3, F, Cl, Br and I substituents at positions 4 and/or 5 and/or 8 of the β-carboline skeleton, wherein X, R1 to R4 are as defined in claim 1.

16. Use of halogenated β-carbolines according to any one of claims 1 to 15, wherein the β-carbolines are used in animal experiments for investigating the causes and pathogenesis of neurodegenerative disorders as well as for studying behavorial changes.

## Revendications

1. Utilisation de β-carbolines répondant à la formule (I) 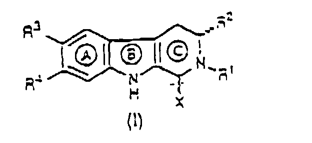 dans laquelle une, deux ou trois liaisons doubles sont présentes dans le noyau C,
X a la signification de -C(Hal)₃, -CH(Hal)₂, -CH₂(Hal) ou =C(Hal)₂, avec Hal = F, Cl, Br ou I, et les substituants R¹, R², R³ et R⁴ ont les significations suivantes :
R¹ : -H, un groupe alkyle en C₁-C₁₈ (par exemple méthyle, éthyle, propyle, n-butyle, isobutyle, tertio-butyle, etc.), -CH₂-CH=CH₂, -CH₂-C₆H₅, un groupe acyle ou aroyle, avec la condition que R¹ puisse être aussi une paire d'électrons libres quand l'azote prend part à la formation d'une liaison double,
R² : -H, -COOH, -COOCH₃, -COOC₂H₅, -COOCH₂-CH=CH₂, -COOCH₂-C₆H₅, -COONH₂, -CO₂NR₂, -CH₂OAc, -CH₂-OH, etc.,
R³ : H-, HO-, CH₃O-, C₂H₅O-, C₆H₅-CH₂O-, CH₃COO-, C₂H₅COO-, etc.,
R⁴ : H-, HO-, CH₃O-, C₂H₅O-, C₆H₅-CH₂O-, CH₃COO-, C₂H₅COO-, etc.,
in vivo chez un modèle animal ou in vitro en tant que substances types pour le développement progressif de maladies neurodegénératives.

2. Utilisation selon la revendication 1, dans laquelle les maladies neurodégénératives sont la. maladie de Parkinson ou la maladie d'Alzheimer.

3. Utilisation selon la revendication 1 ou 2, dans laquelle la β-carboline répond à l'une des formules (1) à (5) suivantes : 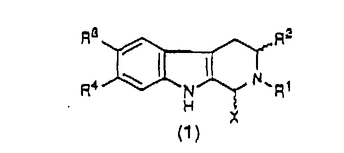 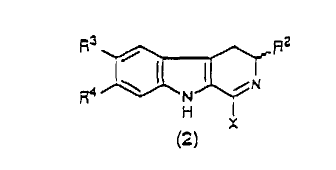 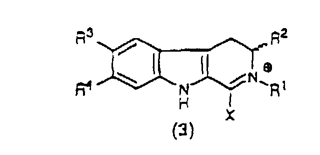 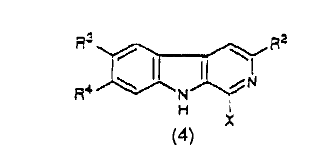 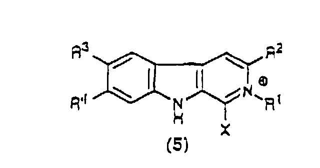 et X et R¹ à R⁴ ont les significations définies dans la revendication 1.

4. Utilisation selon l'une des revendications 1 à 3, dans laquelle, dans la formule (I), R¹ à R⁴ = H, et X = C(Cl)₃ ou C(Br)₃ ou C(F)₃.

5. Utilisation selon l'une des revendications 1 à 3, dans laquelle, dans la formule (I), R¹ = un groupe alkyle en C₁-C₁₈, et R² à R¹ ont les significations définies dans la revendication 1.

6. Utilisation selon l'une des revendications 1 à 3, dans laquelle R² = -COOH, -COOCH₃, -COOC₂H₅, -COOCH₂-CH=CH₂, -COOCH₂-C₆H₅, -COONH₂, -CO₂NR₂, -CH₂OAc ou -CH₂OH, et X, R¹, R³ et R⁴ ont les significations définies dans la revendication 1.

7. Utilisation selon l'une des revendications 1 à 3, dans laquelle R⁴ = H et R³ = H, HO-CH₃O-, C₂H₅O-, C₆H₅-CH₂O-, CH₃COO- ou C₂H₅COO-, et X, R¹ et R² ont les significations définies dans la revendication 1.

8. Utilisation selon l'une des revendications 1 à 3, dans laquelle R³ = H et R⁴ = H, HO-, CH₃O-, C₂H₅O-, C₆H₅-CH₂O-, CH₃COO- ou C₂H₅COO-, et X, R¹ et R² ont les significations définies dans la revendication 1.

9. Utilisation selon l'une des revendications 1 à 3, dans laquelle X est un groupe =C(HAl)₂ avec Hal - F, Cl, Br ou I, et R¹ à R⁴ ont les significations définies dans la revendication 1.

10. Utilisation de β-carbolines halogénées selon la revendication 3, dans laquelle, dans la formule (1), le groupe X est un groupe -C(Cl)₃ un groupe -C(Br)₃ ou un groupe -C(F)₃, et R¹ à R⁴ ont les significations définies dans la revendication 1.

11. Utilisation de β-carbolines halogénées sous la forme d'énantiomères purs selon la revendication 3, dans laquelle, dans la formule (1), le groupe X est un groupe -C(Hal)₃ avec Hal = F, Cl, Br ou , et R² = H, et R¹, R³ et R⁴ ont les significations définies dans la revendication 1.

12. Utilisation de β-carbolines halogénées sous la forme de diastéréomères purs selon la revendication 3, dans laquelle, dans les formules (1) à (3), R² = -COOH, -COOCH₃, -COOC₂H₅, -COOCH₂-CH=CH₂, -COOCH₂-C₆H₅, -COONH₂, -CO₂NR₂, -CH₂OAc ou -CH₂OH, et X, R¹, R³ et R⁴ ont les significations définies dans la revendication 1.

13. Utilisation de β-carbolines halogénées selon la revendication 3, dans laquelle l'atome d'azote dans le noyau indolique est alkylé ou acylé, et X et R¹ à R⁴ ont les significations définies dans la revendication 1.

14. Utilisation de β-carbolines halogénées selon la revendication 3, dans laquelle, dans la formule (1), R¹ = -COOCl₃ ou -COCH₂Cl et -COCH₂Cl, et X et R² à R⁴ ont les significations définies dans la revendication 1.

15. Utilisation de β-carbolines halogénées selon l'une des revendications 1 à 14 avec des substituants OH-, OCH₃-, CH₃-, F-, Cl-, Br- et I aux positions 4 et/ou 5 et/ou 8 du squelette de la β-carboline, X et R¹ à R⁴ ayant les significations définies dans la revendication 1.

16. Utilisation de β-carbolines halogénées selon l'une des revendications 1 à 15, dans laquelle on utilise les β-carbolines dans des essais sur des animaux expérimentaux pour analyser les causes et la pathogénèse de maladies neurodégénératives ainsi que pour étudier des modifications du comportement.
